# EUROPEAN PATENT APPLICATION

(11) **EP 4 714 398 A1**
(43) Date of publication of application: **25.03.2026**
(21) Application number: 24807538.4
(22) Date of filing: 14.05.2024
(51) Int. Cl.: A61F 2/12, A61F 2/00, A61L 27/18, B29C 41/04, B29C 41/14, B29C 41/48, B29C 41/52, B29C 41/38

(54) **BREAST IMPLANT AND MANUFACTURING METHOD FOR SAME**

(30) Priority: 16.05.2023 KR 20230062883; 12.10.2023 KR 20230135885
(71) Applicant: Oh, Hae Seok, Seoul 06627 (KR)
(72) Inventor: HONG, Junghyun, Yeoncheon-gun, Gyeonggi-do 11018 (KR); OH, Hae Seok, Seoul 06627 (KR)
(74) Representative: Buzzi, Notaro & Antonielli d'Oulx S.p.A.
(86) International application number: PCT/KR2024/006578
(87) International publication number: WO 2024/237667

(57) **Abstract**

A breast implant of the present disclosure includes: a first shell having an inner space in which a first filling material is accommodated; a second shell surrounding the first shell and having an inner space in which a second filling material is accommodated; a third shell surrounding the second shell and having an inner space in which a third filling material is accommodated; and an inlet providing a space through which the first filling material, the second filling material, and the third filling material are injected into the first shell, the second shell, and the third shell, respectively.

## Description

### CROSS-REFERENCE TO RELATED APPLICATION

This application is a U.S. Bypass Continuation Application of International Application No. PCT/KR2024/006578, filed on May 14, 2024, which claims priority to and the benefit of Korean Patent Application No. 10-2023-0062883, filed on May 16, 2023, and Korean Patent Application No. 10-2023-0135885, filed on October 12, 2023, the disclosure of which is incorporated herein by reference in its entirety.

### BACKGROUND

### Technical Field

The present invention relates to a breast implant and a method of manufacturing the same, and more particularly, to a breast implant including a plurality of shells and a method of manufacturing the same.

### Background Art

Breast augmentation is currently one of the most commonly performed cosmetic procedure around the world. In addition, about two million or more people are diagnosed with breast cancer worldwide each year, and the number of patients with breast cancer who receive breast reconstruction is also increasing. Accordingly, the breast implant market is gradually growing for purposes such as enhancing the appearance of breasts and reconstructing breasts.

When surgery for inserting a breast implant into the body is performed, an incision of about 5 cm or more has to be made in the skin of a patient due to the volume of the breast implant, and such an incision reduces the patient's satisfaction on the appearance. In addition, the breast implant is provided in a round shape different from the actual breast shape and has a uniform density inside, causing an unnatural texture and reducing the patient's satisfaction. Further, many patients who received breast augmentation or breast reconstruction are mentally stressed about rupture of breast implants in their bodies.

### SUMMARY

### Technical Problem

The present invention is directed to addressing the problems described above.

Specifically, the present invention is directed to providing a breast implant that can reduce the size of an incision scar in patients who receive breast augmentation or breast reconstruction.

The present invention is also directed to providing a breast implant that has a shape and a texture similar to those of a breast made of mammary gland tissue and fat tissue.

The present invention is also directed to providing a breast implant that can reduce mental stress about rupture of breast implants for patients who receive breast augmentation or breast reconstruction.

The present invention is also directed to providing a method of manufacturing a breast implant that has a shape and a texture similar to those of a breast made of mammary gland tissue and fat tissue.

The present invention is also directed to providing a method of manufacturing a breast implant that can reduce the size of an incision scar in patients who receive breast augmentation or breast reconstruction.

The present invention is also directed to providing a method of manufacturing a breast implant that can reduce mental stress about rupture of breast implants for patients who receive breast augmentation or breast reconstruction.

### Technical Solution

To achieve the above objectives, the present disclosure provides a breast implant including: a first shell having an inner space in which a first filling material is accommodated; a second shell surrounding the first shell and having an inner space in which a second filling material is accommodated; a third shell surrounding the second shell and having an inner space in which a third filling material is accommodated; and an inlet providing a space through which the first filling material, the second filling material, and the third filling material are injected into the first shell, the second shell, and the third shell, respectively.

In an exemplary embodiment, a density of the first filling material may be higher than a density of the second filling material, and the density of the second filling material may be higher than a density of the third filling material.

In an exemplary embodiment, a viscosity of the first filling material may be higher than a viscosity of the second filling material, and the viscosity of the second filling material may be higher than a viscosity of the third filling material.

In an exemplary embodiment, a cohesive force of the first filling material may be greater than a cohesive force of the second filling material, and the cohesive force of the second filling material may be greater than a cohesive force of the third filling material.

In an exemplary embodiment, the first filling material may be a first silicone, the second filling material may be a second silicone whose density is lower than the first silicone, and the third filling material may be a third silicone whose density is lower than the second silicone.

In an exemplary embodiment, the first filling material may be a first silicone, the second filling material may be a second silicone whose density is lower than the first silicone, and the third filling material may be a saline solution whose density is lower than the second silicone.

In an exemplary embodiment, the inlet may be provided at a portion where the first shell, the second shell, and the third shell are combined.

In an exemplary embodiment, the inlet may include a first inlet providing a space through which the first filling material is injected into the first shell; a second inlet surrounding the first inlet and providing a space through which the second filling material is injected into the second shell; and a third inlet surrounding the second inlet and providing a space through which the third filling material is injected into the third shell.

In an exemplary embodiment, the inlet may correspond to an areolar portion of the human body.

In an exemplary embodiment, the breast implant may further include a sealing member blocking the inlet.

In an exemplary embodiment, the first shell may include a first filling portion accommodating the first filling material and a first hanging portion connected to the first filling portion and providing the first filling portion in a hanging form inside the second shell, and the second shell may include a second filling portion accommodating the second filling material and a second hanging portion connected to the second filling portion and providing the second filling portion in a hanging form inside the third shell.

The present disclosure provides a breast implant including: a first shell having an inner space in which a first filling material is accommodated; a second shell surrounding the first shell and having an inner space in which a second filling material is accommodated; and an inlet providing a space through which the first filling material and the second filling material are injected into the first shell and the second shell, respectively, wherein a density of the first filling material is higher than a density of the second filling material.

In an exemplary embodiment, the first filling material may be a silicone having a first density, and the second filling material may be a silicone having a second density lower than the first density.

In an exemplary embodiment, the first filling material may be a silicone having a first density, and the second filling material may be a saline solution having a second density lower than the first density.

In an exemplary embodiment, a viscosity of the first filling material may be higher than a viscosity of the second filling material.

In an exemplary embodiment, a cohesive force of the first filling material may be greater than a cohesive force of the second filling material.

In an exemplary embodiment, the inlet may correspond to an areolar portion of the human body, and the inlet may include a first inlet providing a space through which the first filling material is injected into the first shell; and a second inlet surrounding the first inlet and providing a space through which the second filling material is injected into the second shell.

In an exemplary embodiment, the first shell may include a first filling portion accommodating the first filling material and a first hanging portion connected to the first filling portion and providing the first filling portion in a hanging form inside the second shell, wherein, when the breast implant is placed on a floor with the inlet facing upward, a horizontal cross-sectional area of the first hanging portion may be smaller than a horizontal cross-sectional area of the first filling portion.

To achieve the above objectives, an exemplary embodiment of the present disclosure provides a method of manufacturing a breast implant, the method including: coating a surface of a breast implant mold structure including a first mold portion having a first volume, a second mold portion having a second volume smaller than the first volume, and a connecting mold portion connecting the first mold portion and the second mold portion; forming a first breast implant shell including a first shell corresponding to the first mold portion, a second shell corresponding to the second mold portion, and a hanging shell corresponding to the connecting mold portion by hardening a coating liquid on the surface of the breast implant mold structure; separating the breast implant mold structure from the first breast implant shell; and forming a second breast implant shell having a structure in which the second shell is hanging due to the hanging shell in an inner space of the first shell by inserting the second shell and the hanging shell into the inner space of the first shell.

In an exemplary embodiment, the method may further include: closing an opening of the first shell and an opening of the hanging shell by bonding patches; and injecting a first filling material into the first shell and injecting a second filling material into the second shell and the hanging shell.

In an exemplary embodiment, a first density of the first filling material may be lower than a second density of the second filling material.

In an exemplary embodiment, a first viscosity of the first filling material may be lower than a second viscosity of the second filling material.

In an exemplary embodiment, a first cohesive force of the first filling material may be smaller than a second cohesive force of the second filling material.

In an exemplary embodiment, a first elastic modulus of the first filling material may be lower than a second elastic modulus of the second filling material.

In an exemplary embodiment, the first filling material may be a first silicone, and the second filling material may be a second silicone whose density is higher than the first silicone.

In an exemplary embodiment, the second filling material may be silicone, and the first filling material may be a saline solution whose density is lower than the silicone.

In an exemplary embodiment, the method may further include: adjusting a thickness of the coating liquid on the breast implant mold structure by rotating the breast implant mold structure; and checking for leakage from the first breast implant shell by injecting a test liquid into the first breast implant shell.

In an exemplary embodiment, the connecting mold portion of the breast implant mold structure may have a shape whose horizontal width gradually decreases away from the first mold portion and toward the second mold portion.

In an exemplary embodiment, the connecting mold portion of the breast implant mold structure may have a shape whose horizontal width gradually increases away from the first mold portion and toward the second mold portion.

In an exemplary embodiment, the second mold portion may be formed in the shape of a mammary gland tissue including a plurality of shell pockets.

In an exemplary embodiment, a vertical length of the connecting mold portion may range from 0.2 cm to 4.0 cm.

In an exemplary embodiment, a volume of the second shell may range from 25% to 85% of a volume of the first shell.

### [Advantageous Effects]

Since a breast implant of the present disclosure includes a plurality of shells, and the breast implant can be inserted into the body while only some of the plurality of shells are filled with a filling material, the size of an incision scar in a patient can be reduced, and the patient's satisfaction on the appearance can be enhanced.

In addition, since a breast implant of the present disclosure includes a plurality of shells, and an outermost shell among the plurality of shells can include a saline solution as a filling material, when the breast implant is inserted into the body, the size of an incision scar in a patient can be reduced, and the patient's satisfaction on the appearance can be enhanced.

In addition, since a breast implant of the present disclosure includes a plurality of shells, and filling materials with which the plurality of shells are filled can have densities, viscosities, and cohesive forces that gradually increase toward an inner side of the breast implant, the breast implant of the present disclosure can implement a shape and a texture similar to those of the breasts of the body.

In addition, since a shell forming an exterior of a breast implant of the present disclosure can be filled with a saline solution, even when the shell is damaged due to external impact and the filling material leaks, the filling material can be absorbed into the body, and thus the rate of capsular contracture can be reduced, and patients' mental stress about rupture of breast implants can be reduced.

Since a method of manufacturing a breast implant according to an exemplary embodiment of the present disclosure can include forming a first breast implant shell including a first shell, a second shell, and a hanging shell connecting the first shell and the second shell, forming a second breast implant shell having a structure in which the second shell is hanging due to the hanging shell in an inner space of the first shell by inserting the second shell and the hanging shell into the inner space of the first shell, and filling the first shell with a first filling material and filling the second shell and the hanging shell with a second filling material, a breast implant manufactured using the method of manufacturing a breast implant of the present disclosure can implement a shape and a texture similar to those of the breasts made of mammary gland tissue and fat tissue.

In addition, a breast implant manufactured using the method of manufacturing a breast implant according to an exemplary embodiment of the present disclosure can be compressed more easily than existing implants during breast augmentation or breast reconstruction, and since an outer shell portion can be filled after it is placed in the body, the size of an incision scar in patients can be reduced.

Further, when an outer shell portion of a breast implant manufactured using the method of manufacturing a breast implant according to an exemplary embodiment of the present disclosure includes a saline solution, when rupture of the breast implant occurs, a patient can immediately recognize that the rupture has occurred, and the physiological saline solution is more safely absorbed into the body. In this way, patients can safely plan to receive surgery for replacing or removing breast implants. That is, mental stress about rupture of breast implants can be reduced for patients who receive breast augmentation or breast reconstruction.

### BRIEF DESCRIPTION OF THE DRAWINGS

FIG. 1 is a view showing a structure of a breast implant according to an exemplary embodiment of the present disclosure.
FIG. 2 is a view showing a plan view of an inlet of FIG. 1.
FIG. 3 is a view showing the breast implant of the present disclosure that is placed in a breast of a patient who has received breast reconstruction.
FIG. 4 is a view showing the breast implant of the present disclosure that is placed in a breast of a person who has received breast augmentation according to an exemplary embodiment of the present disclosure.
FIG. 5 is a view showing the position of an incision made in the body for breast augmentation or breast reconstruction.
FIG. 6 is a view showing the breast implant in which some of a plurality of shells are filled with a filling material.
FIG. 7 is a view showing a breast into which a breast implant according to a comparative example is inserted.
FIG. 8 is a view showing a breast into which the breast implant of the present disclosure is inserted.
FIG. 9 is a view showing the breast into which the breast implant according to the comparative example is inserted.
FIG. 10 is a view showing the breast into which the breast implant of the present disclosure is inserted.
FIG. 11 is a flowchart showing a flow of a method of manufacturing a breast implant according to an exemplary embodiment of the present disclosure.
FIG. 12 is a flowchart showing a flow of a method of manufacturing a breast implant according to an exemplary embodiment of the present disclosure.
FIG. 13 is a view showing a breast implant according to an exemplary embodiment of the present disclosure.
FIG. 14 is a view showing a structure of a breast implant according to an exemplary embodiment of the present disclosure.
FIG. 15 is a view showing movements of a plurality of shells in the breast implant according to an exemplary embodiment of the present disclosure.
FIG. 16 is a flowchart showing a flow of a breast implant manufacturing method according to an exemplary embodiment of the present disclosure.
FIG. 17 is a view showing coating a surface of a breast implant mold structure according to an exemplary embodiment of the present disclosure.
FIGS. 18 and 19 are enlarged views of a connecting mold portion of the breast implant mold structure that is marked "A" in FIG. 17.
FIG. 20 is a view showing forming a first breast implant shell by hardening a coating liquid on the surface of the breast implant mold structure according to an exemplary embodiment of the present disclosure.
FIG. 21 is a view showing separating the breast implant mold structure from the first breast implant shell (S1) according to an exemplary embodiment of the present disclosure.
FIG. 22 is a view showing forming a second breast implant shell by inserting a second shell and a hanging shell into an inner space of a first shell according to an exemplary embodiment of the present disclosure.
FIG. 23 is a flowchart showing a flow of a breast implant manufacturing method according to an exemplary embodiment of the present disclosure.
FIG. 24 is a view showing closing a first opening of the first shell and a second opening of the hanging shell by bonding patches according to an exemplary embodiment of the present disclosure.
FIG. 25 is a view showing injecting a first filling material into the first shell and injecting a second filling material into the second shell and the hanging shell according to an exemplary embodiment of the present disclosure.
FIG. 26 is a flowchart showing a flow of a breast implant manufacturing method according to an exemplary embodiment of the present disclosure.
FIG. 27 is a view showing coating a surface of a breast implant mold structure according to an exemplary embodiment of the present disclosure.
FIG. 28 is a view showing a method of manufacturing a breast implant using a breast implant mold structure according to an exemplary embodiment of the present disclosure.
FIG. 29 is a view showing a breast implant according to a comparative example and a breast implant of the present disclosure.
FIG. 30 is a view showing shapes of the breast implant according to the comparative example and the breast implant of the present disclosure when they are placed on a flat surface.
FIG. 31 is a view showing shapes of the breast implant according to the comparative example and the breast implant of the present disclosure when they are placed on a vertical surface.

### DETAILED DESCRIPTION

A breast implant of the present disclosure includes: a first shell having an inner space in which a first filling material is accommodated; a second shell surrounding the first shell and having an inner space in which a second filling material is accommodated; a third shell surrounding the second shell and having an inner space in which a third filling material is accommodated; and an inlet providing a space through which the first filling material, the second filling material, and the third filling material are injected into the first shell, the second shell, and the third shell, respectively.

Hereinafter, preferred embodiments of the present disclosure will be described in detail with reference to the accompanying drawings. Advantages and features of the present disclosure and methods of achieving them will be apparent with reference to the embodiments described below in detail with the accompanying drawings. However, the technical idea of the present disclosure is not limited to the embodiments below and can be implemented in various different forms, the embodiments below only make the technical idea of the present disclosure complete and are provided to fully inform those of ordinary skill in the art to which the present disclosure pertains of the scope of the present disclosure, and the technical idea of the present disclosure is only defined by the scope of the claims.

In assigning reference numerals to components of each drawing, it should be noted that like components are denoted by like reference numerals wherever possible even when the components are shown in different drawings. In addition, in describing the present disclosure, when detailed description of a related known configuration or function is determined as having the possibility of obscuring the gist of the present disclosure, the detailed description thereof will be omitted. In addition, reference numerals in FIG. x are construed as denoting components indicated in FIG. x and are not construed as referring to components in another drawing even if the reference numerals in the other drawing are the same as the reference numerals in FIG. x.

Unless defined otherwise, all terms including technical and scientific terms used herein may have the same meaning as commonly understood by those of ordinary skill in the art to which the present disclosure pertains. In addition, terms defined in commonly used dictionaries should not be interpreted in an idealized or overly formal sense unless expressly so defined herein. The terms used herein are for explaining the embodiments and are not intended to limit the present disclosure. In the present specification, a singular expression includes a plural expression unless the context clearly indicates otherwise.

In addition, in describing the components of the present disclosure, terms such as "first," "second," "A," "B," "(a)," and "(b)" may be used. Such terms are only for distinguishing one component from another component, and the essence, sequence, order, or the like of the corresponding component is not limited by the terms. It is to be understood that when a certain component is mentioned as being "connected, " "coupled," or "linked" to another component, the component may be connected or linked directly to the other component, but another component may also be "connected, " "coupled," or "linked" between the two components.

The terms "comprises" and/or "comprising" used herein do not preclude the presence or addition of one or more components, steps, operations, and/or devices other than those mentioned.

A component including a common function with a component included in any one embodiment may be described using the same name in another embodiment. Unless the context clearly indicates otherwise, description made in any one embodiment may apply to another embodiment, and detailed description may be omitted within a redundant range or a range that can be clearly understood by those of ordinary skill in the art.

Hereinafter, the present invention will be described in detail with reference to preferred embodiments of the present invention and the accompanying drawings.

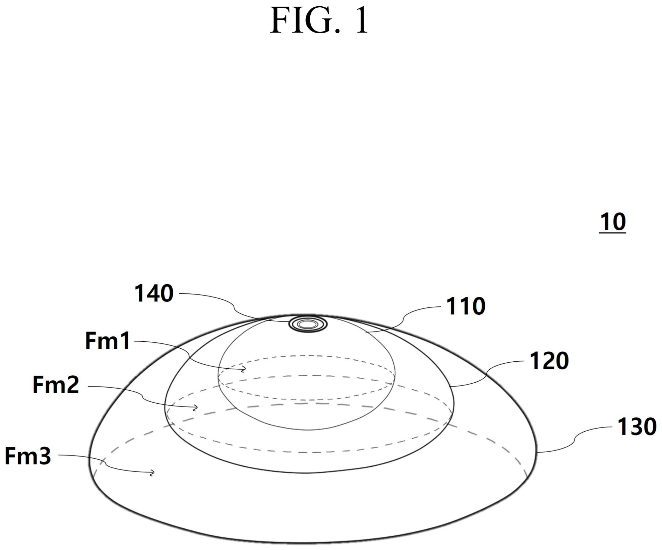

FIG. 1 is a view showing a structure of a breast implant 10 according to an exemplary embodiment of the present disclosure.

Referring to FIG. 1, the breast implant 10 of the present disclosure may include a first shell 110, a second shell 120, a third shell 130, a first filling material Fm1, a second filling material Fm2, a third filling material Fm3, and an inlet 140.

The first shell 110 may be a shell accommodating the first filling material Fm1. Specifically, the first shell 110 may be a shell providing an inner space in which the first filling material Fm1 is accommodated and may be a shell placed at an innermost side among the plurality of shells 110, 120, and 130 included in the breast implant 10 of the present disclosure.

In an exemplary embodiment, a material of the first shell 110 may include silicone. However, the material of the first shell 110 is not limited to silicone and may also include another material. For example, the material of the first shell 110 may include a material such as flexible rubber. In addition, the material of the first shell 110 may include an organic compound or a polymer material having biocompatibility.

The first shell 110 may be placed inside the second shell 120 which will be described below. In an exemplary embodiment, the first shell 110 may be provided in an inner space of the second shell 120 in a state in which at least a portion of the first shell 110 is combined with the second shell 120. For example, at least a portion of the first shell 110 may be directly combined with the second shell 120, or at least a portion of the first shell 110 may be combined with the second shell 120 using a separate combining member.

In other words, at least a portion of the first shell 110 may be combined with the second shell 120, and the first shell may be provided in a hanging state in the inner space of the second shell 120. Accordingly, the portion of the first shell 110 may freely move in the inner space of the second shell 120 based on an external force caused by movement of the body including the breast implant 10 of the present disclosure or based on gravity. For example, compared to a breast implant consisting of a single shell, movement may be easier for the plurality of shells included in the breast implant 10 of the present disclosure.

The first filling material Fm1 may be a material with which an inner space of the first shell 110 is filled. In an exemplary embodiment, a material of the first filling material Fm1 may include silicone. Specifically, the material of the first filling material Fm1 may be silicone provided in a gel form. In addition, the material of the first filling material Fm1 may be a saline solution. However, the material of the first filling material Fm1 is not limited to those mentioned above.

The first filling material Fm1 may have a first density. The density of the first filling material Fm1 may be defined as mass per unit volume of the first filling material Fm1. For example, when the first filling material Fm1 includes a first silicone, a density of the first silicone may be defined as the first density.

In an exemplary embodiment, the first density of the first filling material Fm1 may be higher than a second density of the second filling material Fm2 with which the second shell 120, which will be described below, is filled and may be higher than a third density of the third filling material Fm3 with which the third shell 130, which will be described below, is filled. That is, the first density of the first filling material Fm1 may have the highest value among the densities of the plurality of filling materials Fm1, Fm2, and Fm3.

In addition, the first filling material Fm1 may have a first viscosity. The viscosity of the first filling material Fm1 may be defined as a glutinous, sticky property of the first filling material Fm1, and the viscosity of the first filling material Fm1 may be defined as the degree of viscosity of the first filling material Fm1. For example, a high viscosity may mean relatively high resistance to a fluid flow, and a low viscosity may mean relatively low resistance to a fluid flow.

In an exemplary embodiment, the first viscosity of the first filling material Fm1 may be higher than a second viscosity of the second filling material Fm2 with which the second shell 120, which will be described below, is filled and may be higher than a third viscosity of the third filling material Fm3 with which the third shell 130, which will be described below, is filled. That is, the first viscosity of the first filling material Fm1 may have the highest value among the viscosities of the plurality of filling materials Fm1, Fm2, and Fm3.

For example, a coefficient of viscosity of the first filling material Fm1 may be defined as a numerical value relating to viscosity of a liquid. For example, a first coefficient of viscosity of the first filling material Fm1 may be higher than a second coefficient of viscosity of the second filling material Fm2 with which the second shell 120, which will be described below, is filled and may be higher than a third coefficient of viscosity of the third filling material Fm3 with which the third shell 130, which will be described below, is filled. That is, the first coefficient of viscosity of the first filling material Fm1 may have the highest value among the coefficients of viscosity of the plurality of filling materials Fm1, Fm2, and Fm3.

In addition, the first filling material Fm1 may have a first cohesive force. The cohesive force of the first filling material Fm1 may be defined as an attractive force between molecules constituting the first filling material Fm1. For example, when the first filling material Fm1 includes a first silicone, a cohesive force of the first silicone may be defined as the first cohesive force.

In an exemplary embodiment, the first cohesive force of the first filling material Fm1 may be greater than a second cohesive force of the second filling material Fm2 with which the second shell 120, which will be described below, is filled and may be greater than a third cohesive force of the third filling material Fm3 with which the third shell 130, which will be described below, is filled. That is, the first cohesive force of the first filling material Fm1 may have the greatest value among the cohesive forces of the plurality of filling materials Fm1, Fm2, and Fm3.

The breasts of the body are made up of mammary gland tissue and fat tissue surrounding the mammary gland tissue and thus tend to spread in the horizontal direction, which is perpendicular to the direction of gravity, due to gravity when a person is lying down and tend to drop in the vertical direction, which is parallel to the direction of gravity, due to gravity when the person is standing.

Since the first filling material Fm1 in the first shell 110 of the present disclosure can have the highest density, the highest viscosity, and the greatest cohesive force among the filling materials Fm1, Fm2, and Fm3 with which the plurality of shells 110, 120, and 130 are filled, the breast implant 10 of the present disclosure can implement movement similar to movement of the breasts of the body.

In addition, since the first filling material Fm1 in the first shell 110 of the present disclosure can have the highest density, the highest viscosity, and the greatest cohesive force among the filling materials Fm1, Fm2, and Fm3 with which the plurality of shells 110, 120, and 130 are filled, the breast implant 10 of the present disclosure can implement a texture similar to a texture of the breasts of the body.

The second shell 120 may be a shell accommodating the second filling material Fm2. Specifically, the second shell 120 may be a shell providing an inner space in which the second filling material Fm2 is accommodated and may be a shell placed outside the first shell 110 and inside the third shell 130.

In an exemplary embodiment, a material of the second shell 120 may include silicone. However, the material of the second shell 120 is not limited to silicone and may also include another material. For example, the material of the second shell 120 may include a material such as flexible rubber. In addition, the material of the second shell 120 may include an organic compound or a polymer material having biocompatibility.

The second shell 120 may be placed inside the third shell 130 which will be described below. In an exemplary embodiment, the second shell 120 may be provided in an inner space of the third shell 130 in a state in which at least a portion of the second shell 120 is combined with the third shell 130. For example, at least a portion of the second shell 120 may be directly combined with the third shell 130, or at least a portion of the second shell 120 may be combined with the third shell 130 using a separate combining member.

In other words, at least a portion of the second shell 120 may be combined with the third shell 130, and the second shell 120 may be provided in a hanging state in the inner space of the third shell 130. Accordingly, the portion of the second shell 120 may freely move in the inner space of the third shell 130 based on an external force caused by movement of the body including the breast implant 10 or based on gravity. For example, compared to a breast implant consisting of a single shell, movement may be easier for the plurality of shells included in the breast implant 10 of the present disclosure.

When a breast implant according to a comparative example only includes a first shell and a second shell surrounding the first shell (that is, only includes two shells), after the breast implant is inserted into the human body, the second shell placed at an outermost portion may have relatively low mobility due to coming into contact with the body, and the first shell inside the second shell may have relatively high mobility based on an external force caused by movement of the body or based on gravity.

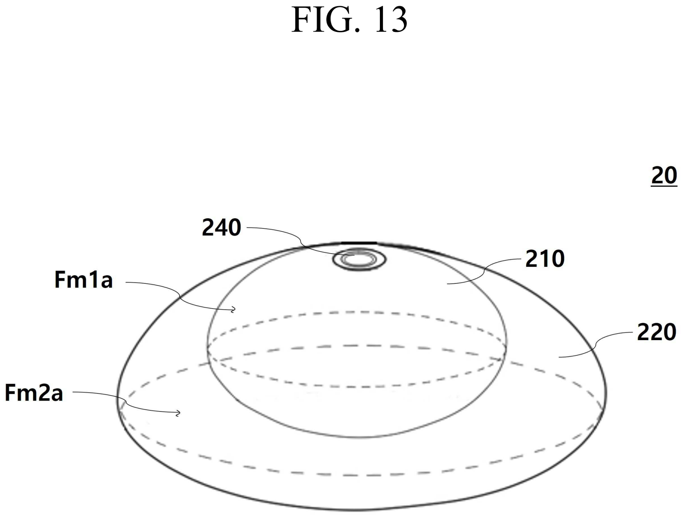

The present disclosure provides the breast implant 10 including the plurality of shells having relatively high mobility in the breasts of the body, thereby implementing freer movement of the breasts and a natural texture of the breasts. That is, compared to the breast implant consisting of two shells, the breast implant of the present disclosure includes three or more shells to secure mobility of the shells placed therein, and the densities, viscosities, and cohesive forces of the filling materials with which the shells are filled are formed to be different from one another, and in this way, the breast implant of the present disclosure can implement freer movement of the breasts and a natural texture of the breasts. However, the present disclosure is not limited thereto and may also provide a breast implant 20 (see FIG. 13) that includes two shells. The breast implant 10 of the present disclosure may include the third shell 130, the second shell 120 placed inside the third shell 130, and the first shell 110 placed inside the second shell 120. Accordingly, even when the breast implant 10 of the present disclosure is inserted into the human body, and the third shell 130 placed at an outermost portion has relatively low mobility due to coming into contact with the body, relatively high mobility based on an external force caused by movement of the body or based on gravity can be secured for the second shell 120 placed inside the third shell 130, and relatively high mobility based on an external force caused by movement of the body or based on gravity can also be secured for the first shell 110 placed inside the second shell 120. Accordingly, the breast implant 10 of the present disclosure can implement free movement of breasts according to movement of the body and gravity.

In addition, since the densities, viscosities, and cohesive forces of the filling materials Fml, Fm2, and Fm3 with which the plurality of shells 110, 120, and 130 of the present disclosure are filled can gradually increase toward an inner side of the breast implant 10 (that is, gradually decrease toward an outer side of the breast implant 10), the breast implant 10 of the present disclosure can not only implement free movement of breasts according to movement of the body and gravity, but also implement a texture similar to the texture of the breasts.

In addition, due to differences in shapes and sizes of mammary gland tissue and fat tissue, the texture and shape of breasts may vary in patients who receive breast augmentation or breast reconstruction. The breast implant 10 of the present disclosure may include three or more shells 110, 120, and 130, and the densities, viscosities, and cohesive forces of the plurality of filling materials Fm1, Fm2, and Fm3 with which the plurality of shells 110, 120, and 130 are filled may be individually determined according to states of breasts (for example, the shape, size, texture, and the like of the breasts) of each patient. In other words, materials of the plurality of filling materials Fm1, Fm2, and Fm3 with which the plurality of shells 110, 120, and 130 of the present disclosure are filled may be freely determined according to the states of breasts of each patient.

The second filling material Fm2 may be a material with which the inner space of the second shell 120 is filled. In an exemplary embodiment, a material of the second filling material Fm2 may include silicone. Specifically, the material of the second filling material Fm2 may be silicone provided in a gel form. In addition, the material of the second filling material Fm2 may be a saline solution. However, the material of the second filling material Fm2 is not limited to those mentioned above.

The second filling material Fm2 may have a second density. The density of the second filling material Fm2 may be defined as mass per unit volume of the second filling material Fm2. For example, when the second filling material Fm2 includes a second silicone, a density of the second silicone may be defined as the second density.

In an exemplary embodiment, the second density of the second filling material Fm2 may be lower than the first density of the first filling material Fm1 with which the first shell 110 is filled and may be higher than the third density of the third filling material Fm3 with which the third shell 130 is filled. That is, the second density of the second filling material Fm2 may have an intermediate value among the densities of the plurality of filling materials Fm1, Fm2, and Fm3.

In addition, the second filling material Fm2 may have a second viscosity. The viscosity of the second filling material Fm2 may be defined as a glutinous, sticky property of the second filling material Fm2, and the viscosity of the second filling material Fm2 may be defined as the degree of viscosity of the second filling material Fm2.

In an exemplary embodiment, the second viscosity of the second filling material Fm2 may be lower than the first viscosity of the first filling material Fm1 with which the first shell 110 is filled and may be higher than the third viscosity of the third filling material Fm3 with which the third shell 130 is filled. That is, the second viscosity of the second filling material Fm2 may have an intermediate value among the viscosities of the plurality of filling materials Fm1, Fm2, and Fm3.

For example, a coefficient of viscosity of the second filling material Fm2 may be defined as a numerical value relating to viscosity of a liquid. For example, a second coefficient of viscosity of the second filling material Fm2 may be lower than the first coefficient of viscosity of the first filling material Fm1 with which the first shell 110 is filled and may be higher than the third coefficient of viscosity of the third filling material Fm3 with which the third shell 130 is filled. That is, the second coefficient of viscosity of the second filling material Fm2 may have an intermediate value among the coefficients of viscosity of the plurality of filling materials Fm1, Fm2, and Fm3.

The second filling material Fm2 may have a second cohesive force. The cohesive force of the second filling material Fm2 may be defined as an attractive force between molecules constituting the second filling material Fm2. For example, when the second filling material Fm2 includes a second silicone, a cohesive force of the second silicone may be defined as the second cohesive force.

In an exemplary embodiment, the second cohesive force of the second filling material Fm2 may be smaller than the first cohesive force of the first filling material Fm1 with which the first shell 110 is filled and may be greater than the third cohesive force of the third filling material Fm3 with which the third shell 130 is filled. That is, the second cohesive force of the second filling material Fm2 may have an intermediate value among the cohesive forces of the plurality of filling materials Fm1, Fm2, and Fm3.

The third shell 130 may be a shell accommodating the third filling material Fm3. Specifically, the third shell 130 may be a shell providing an inner space in which the third filling material Fm3 is accommodated and may be a shell placed at an outermost side among the plurality of shells 110, 120, and 130 included in the breast implant 10 of the present disclosure.

In an exemplary embodiment, a material of the third shell 130 may include silicone. However, the material of the third shell 130 is not limited to silicone and may also include another material. For example, the material of the third shell 130 may include a material such as flexible rubber. In addition, the material of the third shell 130 may include an organic compound or a polymer material having biocompatibility.

The third shell 130 may form an outer surface of the breast implant 10 of the present disclosure. In an exemplary embodiment, since the second shell 120 is accommodated in the inner space of the third shell 130, and the first shell 110 is accommodated in the inner space of the second shell 120, when the breast implant 10 of the present disclosure is observed by visual inspection, only the third shell 130 may be observed, and the first shell 110 and the second shell 120 may not be observed.

In an exemplary embodiment, as at least a portion of the first shell 110 is combined with the second shell 120 and provided in a hanging state, and at least a portion of the second shell 120 is combined with the third shell 130 and provided in a hanging state, the first shell 110 and the second shell 120 of the present disclosure may freely move inside the third shell 130 based on an external force caused by movement of the body including the breast implant 10 or based on gravity. That is, compared to a breast implant consisting of a single shell, movement may be improved for the plurality of shells of the breast implant of the present disclosure.

The third filling material Fm3 may be a material with which the inner space of the third shell 130 is filled. In an exemplary embodiment, a material of the third filling material Fm3 may include silicone. Specifically, the material of the third filling material Fm3 may be silicone provided in a gel form. In addition, the material of the third filling material Fm3 may be a saline solution. However, the material of the third filling material Fm3 is not limited to those mentioned above.

The third filling material Fm3 may have a third density. The density of the third filling material Fm3 may be defined as mass per unit volume of the third filling material Fm3. For example, when the third filling material Fm3 includes a third silicone, a density of the third silicone may be defined as the third density.

In an exemplary embodiment, the third density of the third filling material Fm3 may be lower than the first density of the first filling material Fm1 with which the first shell 110 is filled and may be lower than the second density of the second filling material Fm2 with which the second shell 120 is filled. That is, the third density of the third filling material Fm3 may have the lowest value among the densities of the plurality of filling materials Fm1, Fm2, and Fm3.

In addition, the third filling material Fm3 may have a third viscosity. The viscosity of the third filling material Fm3 may be defined as a glutinous, sticky property of the third filling material Fm3, and the viscosity of the third filling material Fm3 may be defined as the degree of viscosity of the third filling material Fm3.

In an exemplary embodiment, the third viscosity of the third filling material Fm3 may be lower than the first viscosity of the first filling material Fm1 with which the first shell 110 is filled and may be lower than the second viscosity of the second filling material Fm2 with which the second shell 120 is filled. That is, the third viscosity of the third filling material Fm3 may have the lowest value among the viscosities of the plurality of filling materials Fm1, Fm2, and Fm3.

For example, a coefficient of viscosity of the third filling material Fm3 may be defined as a numerical value relating to viscosity of a liquid. For example, a third coefficient of viscosity of the third filling material Fm3 may be lower than the first coefficient of viscosity of the first filling material Fm1 with which the first shell 110 is filled and may be lower than the second coefficient of viscosity of the second filling material Fm2 with which the second shell 120 is filled. That is, the third coefficient of viscosity of the third filling material Fm3 may have the lowest value among the coefficients of viscosity of the plurality of filling materials Fm1, Fm2, and Fm3.

The third filling material Fm3 may have a third cohesive force. The cohesive force of the third filling material Fm3 may be defined as an attractive force between molecules constituting the third filling material Fm3. For example, when the third filling material Fm3 includes a third silicone, a cohesive force of the third silicone may be defined as the third cohesive force.

In an exemplary embodiment, the third cohesive force of the third filling material Fm3 may be smaller than the first cohesive force of the first filling material Fm1 with which the first shell 110 is filled and may be smaller than the second cohesive force of the second filling material Fm2 with which the second shell 120 is filled. That is, the third cohesive force of the third filling material Fm3 may have the smallest value among the cohesive forces of the plurality of filling materials Fm1, Fm2, and Fm3.

As described above, the breasts of the body are made up of mammary gland tissue and fat tissue surrounding the mammary gland tissue and thus tend to spread in the horizontal direction, which is perpendicular to the direction of gravity, due to gravity when a person is lying down and tend to drop in the vertical direction, which is parallel to the direction of gravity, due to gravity when the person is standing.

Since the breast implant 10 of the present disclosure includes the plurality of shells 110, 120, and 130, and the filling materials Fm1, Fm2, and Fm3 with which the plurality of shells 110, 120, and 130 are filled can have densities, viscosities, and cohesive forces that gradually increase toward an inner side of the breast implant 10, the breast implant 10 of the present disclosure can implement movement similar to movement of the breasts of the body.

In addition, since the filling materials Fm1, Fm2, and Fm3 with which the plurality of shells 110, 120, and 130 of the present disclosure are filled can have densities, viscosities, and cohesive forces that gradually increase toward an inner side of the breast implant 10, the breast implant 10 of the present disclosure can implement a texture similar to a texture of the breasts of the body.

In an exemplary embodiment, the plurality of shells 110, 120, and 130 may be provided in a shape similar to a semispherical shape or an elliptical spherical shape. When the breast implant 10 illustrated in FIG. 1 is placed on a floor with the inlet 140 facing upward, the third shell 130 may be provided in a tapered shape in which a horizontal cross-sectional area thereof gradually decreases upward. At this time, a horizontal cross-section of the third shell 130 may have a circular shape or an elliptical shape.

In addition, when the breast implant 10 of the present disclosure is placed on a floor with the inlet 140 facing upward, the first shell 110 and the second shell 120 placed inside the third shell 130 may be provided in a semispherical shape or an elliptical spherical shape in which a horizontal cross-sectional area thereof gradually increases upward due to gravity and then gradually decreases from a predetermined section.

In an exemplary embodiment, a volume of the first shell 110 may range from about 10% to about 25% of the overall volume of the breast implant 10. In addition, a volume of the second shell 120 may range from about 25% to about 30% of the overall volume of the breast implant 10. Further, a volume of the third shell 130 may range from about 45% to about 65% of the overall volume of the breast implant 10. However, the volume ratio of the first to third shells 110, 120, and 130 is not limited to the numerical values mentioned above.

The inlet 140 may provide a space through which the filling materials Fm1, Fm2, and Fm3 are injected into the plurality of shells 110, 120, and 130, respectively. In an exemplary embodiment, the inlet 140 may be provided at portions where the plurality of shells 110, 120, and 130 are combined with one another (that is, come into contact with one another). For example, when the breast implant 10 is placed on a floor, the inlet 140 may be provided at an uppermost end of the breast implant 10 of the present disclosure.

In addition, the breast implant 10 of the present disclosure may be provided in a shape similar to the shape of the breasts of the human body. At this time, the inlet 140 of the breast implant 10 may correspond to an areolar portion of the human body or a portion adjacent to the areolar portion. In the present specification, "the inlet 140 of the breast implant 10 corresponds to an areolar portion of the human body" may mean that the inlet 140 corresponds to a portion where the areola is formed or a portion where subareolar tissue (for example, subareolar mammary gland tissue) is placed. For example, the inlet 140 of the breast implant 10 may correspond to an areolar portion of the human body and may be placed at a portion adjacent to subareolar tissue (for example, subareolar mammary gland tissue).

That is, when the breast implant 10 is inserted into the human body, the inlet 140 of the breast implant 10 may be provided at an areolar portion of the human body or a portion adjacent to the areolar portion.

An areolar portion has a relatively darker color than other portions of the body skin. After the breast implant 10 is inserted into the human body, control of injection or suction of the filling materials Fm1, Fm2, and Fm3 inside the plurality of shells 110, 120, and 130 may be necessary in some cases due to various causes such as discomfort and pain of a patient.

At this time, since the inlet 140 of the breast implant 10 can be present at an areolar portion of the human body or a portion adjacent to the areolar portion, even when an incision is made in a portion of the areolar portion or a portion adjacent to the areolar portion on the skin to control the filling materials Fm1, Fm2, and Fm3 in the breast implant 10, visibility of a scar can be minimized due to a dark color of the areolar portion.

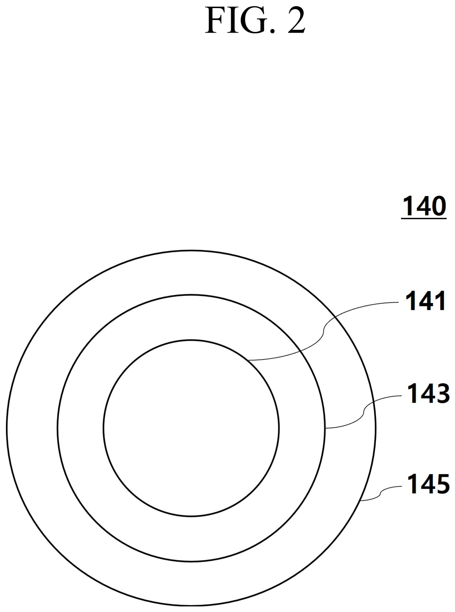

A structure of the inlet 140 of the breast implant 10 of the present disclosure will be described in more detail with reference to FIG. 2.

Although the breast implant 10 of the present disclosure is illustrated as including the three shells 110, 120, and 130, the present disclosure is not limited thereto, and the breast implant 10 of the present disclosure may also include four or more shells.

FIG. 2 is a view showing a plan view of the inlet 140 of FIG. 1.

Referring to FIG. 2, the inlet 140 of the breast implant 10 of the present disclosure may include a first inlet 141, a second inlet 143, and a third inlet 145.

The first inlet 141 may be a space provided to inject the first filling material Fm1 into the first shell 110. In an exemplary embodiment, the first inlet 141 may be placed on an innermost side among the plurality of inlets 141, 143, and 145. For example, the first inlet 141 may be provided in a circular shape.

The second inlet 143 may be a space provided to inject the second filling material Fm2 into the second shell 120. For example, the second inlet 143 may be provided in a ring shape that surrounds the first inlet 141.

In an exemplary embodiment, the second inlet 143 may be placed at an intermediate portion among the plurality of inlets 141, 143, and 145. That is, the second inlet 143 may be placed outside the first inlet 141 and inside the third inlet 145.

The third inlet 145 may be a space provided to inject the third filling material Fm3 into the third shell 130. In an exemplary embodiment, the third inlet 145 may be placed on an outermost side among the plurality of inlets 141, 143, and 145. For example, the third inlet 145 may be provided in a ring shape that surrounds the second inlet 143.

In an exemplary embodiment, when the plurality of shells 110, 120, and 130 are filled with the filling materials Fm1, Fm2, and Fm3 through the plurality of first to third inlets 141, 143, and 145, the plurality of first to third inlets 141, 143, and 145 may be blocked by a sealing member.

In an exemplary embodiment, the sealing member may include an adhesive label that is easy to adhere to the plurality of shells 110, 120, and 130. For example, the sealing member may include a sticker-type adhesive label that is provided to have an area wider than the plurality of first to third inlets 141, 143, and 145 and blocks the first to third inlets 141, 143, and 145. However, the type of sealing member is not limited to that mentioned above.

In an exemplary embodiment, the sealing member may include a silicone material. For example, the sealing member may be a sticker-type adhesive label that is made of a silicone material. However, the material of the sealing member is not limited to that mentioned above.

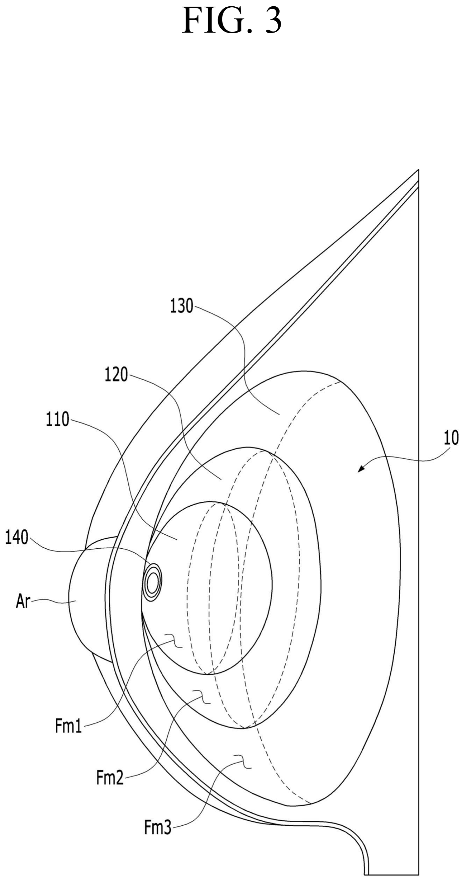

FIG. 3 is a view showing the breast implant 10 of the present disclosure that is placed in a breast of a patient who has received breast reconstruction.

Generally, when patients with breast cancer receive mastectomy, mammary gland tissue of the breast may be removed. Accordingly, patients who received mastectomy often receive breast reconstruction in which a new breast similar to the actual breast is made using a breast implant.

Referring to FIG. 3, the breast implant 10 of the present disclosure may be inserted into a breast of a patient. Specifically, the breast implant 10 of the present disclosure may be inserted into the breast of the patient so that the inlet 140 of the breast implant 10 of the present disclosure corresponds to an areolar portion Ar of the patient.

Since the inlet 140 of the breast implant 10 can be placed to correspond to a portion adjacent to an areolar portion ar of the human body, even when an incision is made in a portion of the areolar portion Ar on the skin to control the filling materials Fm1, Fm2, and Fm3 in the breast implant 10, visibility of a scar can be minimized due to a dark color of the areolar portion.

In an exemplary embodiment, the third shell 130 of the breast implant 10 may form an overall exterior of a breast. For example, a filling amount of the third filing material Fm3 may be determined based on a desired size and shape of a breast of a patient, and the third shell 130 may form an exterior of a breast as the third shell 130 is filled with the third filling material Fm3.

In addition, as the first shell 110 and the second shell 120 are filled with the first filling material Fm1 and the second filling material Fm2, respectively, the first shell 110 and the second shell 120 may freely move in the inner space of the third shell 130 based on an external force caused by movement of the body or based on gravity. That is, compared to a breast implant consisting of a single shell, movement may be improved for the plurality of shells of the breast implant of the present disclosure.

In an exemplary embodiment, the first filling material Fm1 and the second filling material Fm2 with which the first shell 110 and the second shell 120 are filled may be silicone, and the third filling material Fm3 with which the third shell 130 is filled may be a saline solution. Since the third shell 130 forming the exterior of the breast implant 10 of the present disclosure can be filled with a saline solution, even when the third shell 130 is damaged due to external impact and the third filling material Fm3 leaks, the third filling material Fm3 may be absorbed into the body, and the rate of capsular contracture can be reduced. In addition, patients' mental stress about rupture of breast implants can be reduced.

In an exemplary embodiment, the density, viscosity, and cohesive force of the saline solution with which the third shell 130 is filled may be lower and smaller than the density, viscosity, and cohesive force of the silicone with which each of the first shell 110 and the second shell 120 is filled. In addition, the density of the silicone with which the second shell 120 is filled may be lower than the density of the silicone with which the first shell 110 is filled.

That is, since the breast implant 10 of the present disclosure includes the plurality of shells 110, 120, and 130, and the filling materials Fm1, Fm2, and Fm3 with which the plurality of shells 110, 120, and 130 are filled can have densities, viscosities, and cohesive forces that gradually increase toward an inner side of the breast implant 10, the breast implant 10 of the present disclosure can implement movement and a texture similar to those of the breasts of the body.

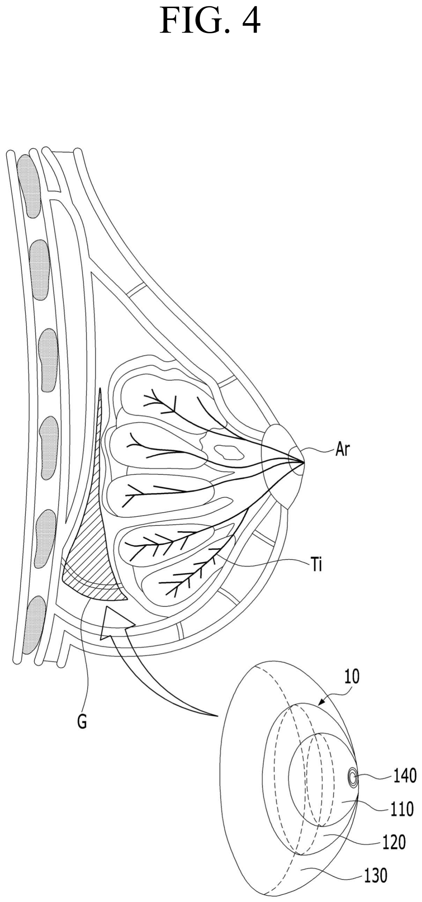

FIG. 4 is a view showing the breast implant 10 of the present disclosure that is placed in a breast of a person who has received breast augmentation according to an exemplary embodiment of the present disclosure.

Unlike a patient who receives breast reconstruction through breast cancer surgery, a person who receives breast augmentation for a cosmetic purpose or the like may have a mammary gland tissue Ti. Referring to FIG. 4, the breast implant 10 of the present disclosure may be placed in a space G provided on an inner side of the mammary gland tissue Ti. Specifically, the breast implant 10 of the present disclosure may be inserted into the space G provided on the inner side of the mammary gland tissue Ti so that the inlet 140 of the breast implant 10 of the present disclosure corresponds to the areolar portion Ar of the patient.

However, the present disclosure is not limited thereto, and the breast implant 10 may be inserted into any other layer in the body. For example, the breast implant 10 may be inserted into a subfascial layer or a half subfascial layer.

Since the inlet 140 of the breast implant 10 can be placed to correspond to a portion adjacent to the areolar portion ar of the human body, even when an incision is made in a portion of the areolar portion Ar on the skin to control the filling materials Fm1, Fm2, and Fm3 in the breast implant 10, visibility of a scar can be minimized due to a dark color of the areolar portion.

In an exemplary embodiment, the third shell 130 of the breast implant 10 may control the size and shape of the exterior of a breast. For example, a filling amount of the third filing material Fm3 may be determined based on a desired size and shape of a breast of a patient, and the third shell 130 may form an exterior of a breast as the third shell 130 is filled with the third filling material Fm3.

In addition, as the first shell 110 and the second shell 120 are filled with the first filling material Fm1 and the second filling material Fm2, respectively, the first shell 110 and the second shell 120 may freely move in the inner space of the third shell 130 based on an external force caused by movement of the body or based on gravity. That is, compared to a breast implant consisting of a single shell, movement may be improved for the plurality of shells of the breast implant of the present disclosure.

In an exemplary embodiment, the first filling material Fm1 and the second filling material Fm2 with which the first shell 110 and the second shell 120 are filled may be silicone, and the third filling material Fm3 with which the third shell 130 is filled may be a saline solution. Since the third shell 130 forming the exterior of the breast implant 10 of the present disclosure can be filled with a saline solution, even when the third shell 130 is damaged due to external impact and the third filling material Fm3 leaks, the third filling material Fm3 may be absorbed into the body, and the rate of capsular contracture can be reduced. In addition, patients' mental stress about rupture of breast implants can be reduced.

In an exemplary embodiment, the density, viscosity, and cohesive force of the saline solution with which the third shell 130 is filled may be lower and smaller than the density, viscosity, and cohesive force of the silicone with which each of the first shell 110 and the second shell 120 is filled. In addition, the density of the silicone with which the second shell 120 is filled may be lower than the density of the silicone with which the first shell 110 is filled.

That is, since the breast implant 10 of the present disclosure includes the plurality of shells 110, 120, and 130, and the filling materials Fml, Fm2, and Fm3 with which the plurality of shells 110, 120, and 130 are filled can have densities, viscosities, and cohesive forces that gradually increase toward an inner side of the breast implant 10, the breast implant 10 of the present disclosure can implement movement and a texture similar to those of the breasts of the body.

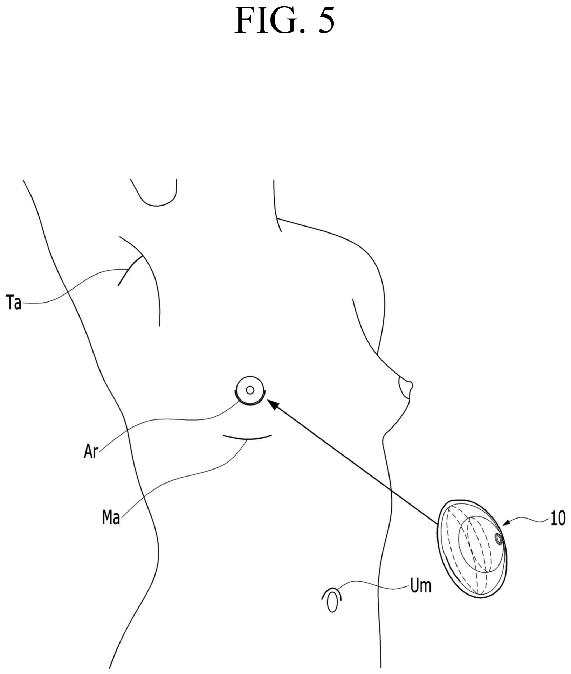

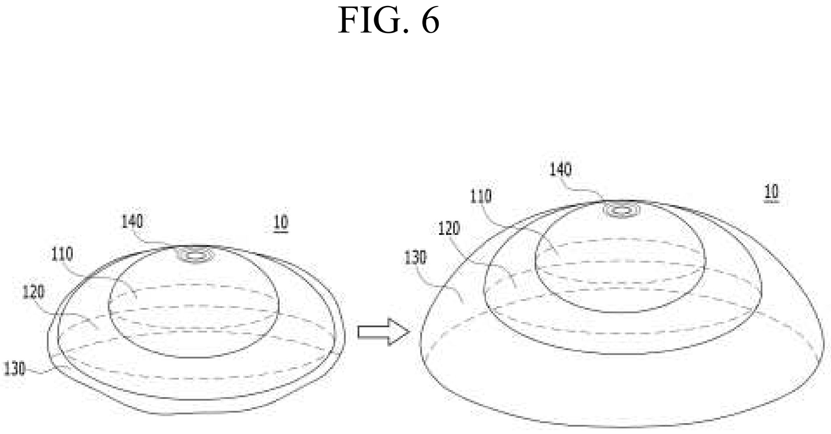

FIG. 5 is a view showing the position of an incision made in the body for breast augmentation or breast reconstruction. In addition, FIG. 6 is a view showing the breast implant 10 in which some of the plurality of shells are filled with a filling material.

Referring to FIG. 5, an incision may be made in at least one of an axillary tail portion Ta, an areolar portion Ar, a submammary portion Ma, and an umbilical portion Um of the body for breast augmentation or breast reconstruction.

When surgery for inserting a typical breast implant into the human body is performed, an incision of about 5 cm or more has to be made in the skin of a patient due to the volume of the breast implant. Such an incision made in the body may reduce the patient's satisfaction on the appearance.

Referring to FIG. 6, for performing breast augmentation or breast reconstruction, the first shell 110 and the second shell 120 of the breast implant 10 of the present disclosure may be provided in a state in which the first shell 110 and the second shell 120 are filled with the first filling material Fm1 and the second filling material Fm2, respectively, and the third shell 130 may be provided in a state in which the third shell 130 is not filled with the third filling material Fm3. The first filling material Fm1 and the second filling material Fm2 may be silicone.

In an exemplary embodiment, in performing breast augmentation or breast reconstruction, after the breast implant 10 in a state in which the first shell 110 and the second shell 120 are filled with the first filling material Fm1 and the second filling material Fm2, respectively, is inserted into the body of a patient, the third shell 130 may be filled with the third filling material Fm3 through the inlet 140.

Since the breast implant 10 of the present disclosure includes the plurality of shells 110, 120, and 130, and the breast implant 10 can be inserted into the body in a state in which only some shells 110 and 120 of the plurality of shells 110, 120, and 130 are filled with the filling materials Fm1 and fm2, the volume (that is, the size) of the breast implant 10 of the present disclosure at the time of inserting the breast implant 10 into the human body can be reduced. Accordingly, a range of an incision made in the skin for breast augmentation or breast reconstruction can be reduced, and the patient's satisfaction on the appearance can be enhanced.

However, the present disclosure is not limited thereto, and the breast implant 10 may be inserted into the body in a state in which all of the plurality of shells 110, 120, and 130 are filled with the filling materials Fm1, Fm2, and Fm3, respectively. At this time, since the breast implant 10 can be inserted into the body in a state in which the outermost shell 130 is filled with a saline solution, the size of an incision scar in the patient can be reduced, and the patient's satisfaction on the appearance can be enhanced.

In addition, as described above, since the breast implant 10 can be placed in the body so that the inlet 140 of the present disclosure corresponds to a portion adjacent to the areolar portion ar of the human body, even when an incision is made in a portion of the areolar portion Ar on the skin to fill the third shell 130 with the third filling material Fm3 through the inlet 140, visibility of a scar can be minimized due to a dark color of the areolar portion Ar.

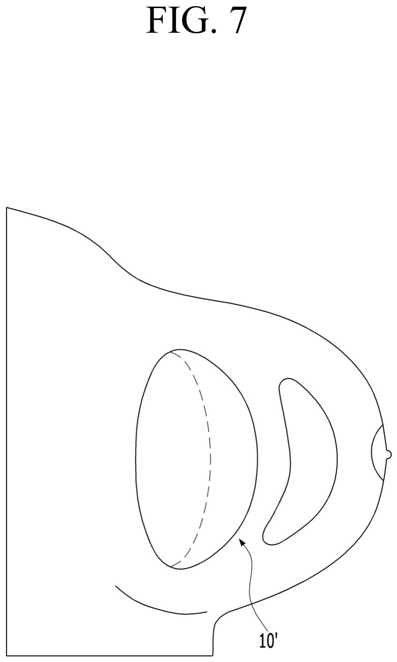

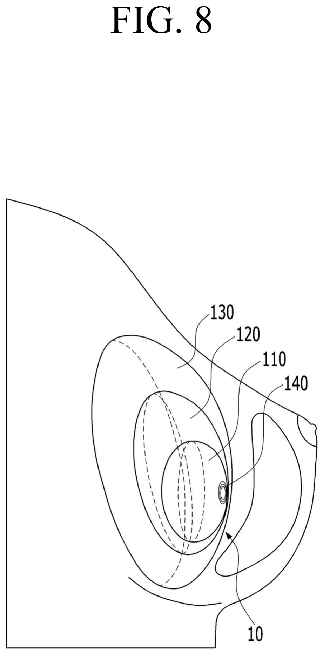

FIG. 7 is a view showing a breast into which a breast implant 10' according to a comparative example is inserted. In addition, FIG. 8 is a view showing a breast into which the breast implant 10 of the present disclosure is inserted.

Referring to FIG. 7, the breast implant 10' according to the comparative example may be an implant including one shell and a filling material with which the shell is filled. The breast implant 10' according to the comparative example consists of one shell, making it difficult to implement the shape of an actual breast, and the inside of the shell has a uniform density, causing an unnatural texture and reducing the patient's satisfaction.

The breasts of the body are each made up of mammary gland tissue and fat tissue. Specifically, an inner portion of each breast has a relatively high density due to mammary gland tissue or the like, and an outer portion of each breast has a relatively low density due to fat tissue. Accordingly, the breasts tend to drop in the vertical direction parallel to the direction of gravity due to gravity when a person is standing. Since the inside of the shell of the breast implant 10' according to the comparative example has a uniform density, the breast into which the breast implant 10' according to the comparative example is inserted does not drop downward despite gravity, and a natural breast shape is not able to be implemented.

Referring to FIG. 8, since the breast implant 10 of the present disclosure includes the plurality of shells 110, 120, and 130, and the filling materials Fm1, Fm2, and Fm3 with which the plurality of shells 110, 120, and 130 are filled can have densities, viscosities, and cohesive forces that gradually increase toward an inner side of the breast implant 10, the breast implant 10 of the present disclosure can implement movement similar to movement of the breasts of the body. That is, when a person with the breast implant 10 of the present disclosure is standing, the plurality of shells 110, 120, and 130 of the breast implant 10 of the present disclosure may drop in the direction parallel to the direction of gravity due to gravity, and a natural breast shape can be implemented.

In addition, since the filling materials Fm1, Fm2, and Fm3 with which the plurality of shells 110, 120, and 130 of the present disclosure are filled can have densities, viscosities, and cohesive forces that gradually increase toward an inner side of the breast implant 10, the breast implant 10 of the present disclosure can implement a texture similar to a texture of the breasts of the body.

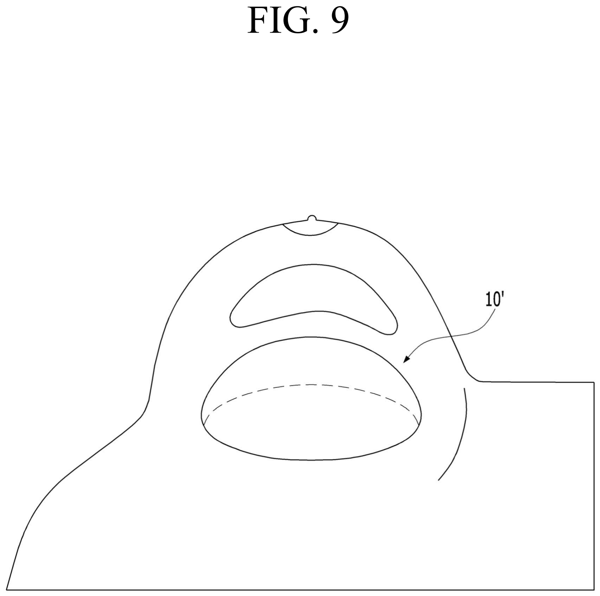

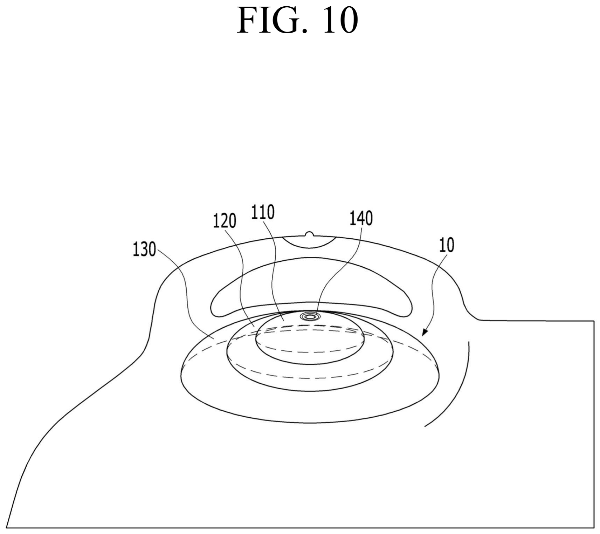

FIG. 9 is a view showing the breast into which the breast implant 10' according to the comparative example is inserted. In addition, FIG. 10 is a view showing the breast into which the breast implant 10 of the present disclosure is inserted.

Referring to FIG. 9, the breast implant 10' according to the comparative example may be an implant including one shell and a filling material with which the shell is filled. The breast implant 10' according to the comparative example consists of one shell, making it difficult to implement the shape of an actual breast, and the inside of the shell has a uniform density, causing an unnatural texture and reducing the patient's satisfaction.

When a person is lying down, breasts tend to spread in a direction perpendicular to the direction of gravity due to gravity. Since the inside of the shell of the breast implant according to the comparative example has a uniform density, the breast into which the breast implant according to the comparative example is inserted does not spread in the horizontal direction despite gravity, and a natural breast shape is not able to be implemented.

Referring to FIG. 10, since the breast implant 10 of the present disclosure includes the plurality of shells 110, 120, and 130, and the filling materials Fm1, Fm2, and Fm3 with which the plurality of shells 110, 120, and 130 are filled can have densities, viscosities, and cohesive forces that gradually increase toward an inner side of the breast implant 10, the breast implant 10 of the present disclosure can implement movement similar to movement of the breasts of the body. That is, when a person with the breast implant 10 of the present disclosure is lying down, the plurality of shells 110, 120, and 130 of the breast implant 10 of the present disclosure may spread in the direction perpendicular to the direction of gravity due to gravity, and a natural breast shape can be implemented.

In addition, since the filling materials Fm1, Fm2, and Fm3 with which the plurality of shells 110, 120, and 130 of the present disclosure are filled can have densities, viscosities, and cohesive forces that gradually increase toward an inner side of the breast implant 10, the breast implant 10 of the present disclosure can implement a texture similar to a texture of the breasts of the body.

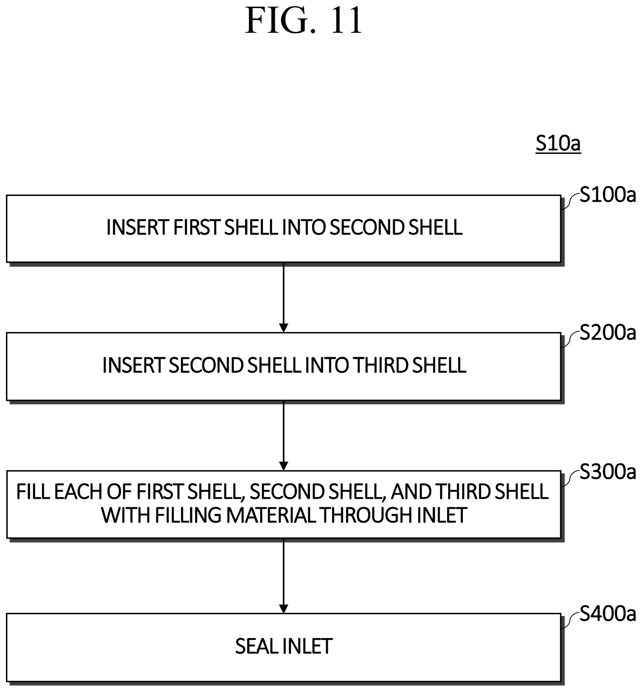

FIG. 11 is a flowchart showing a flow of a method S10a of manufacturing the breast implant 10 according to an exemplary embodiment of the present disclosure.

Referring to FIG. 11, the method S10a of manufacturing the breast implant 10 of the present disclosure may include inserting the first shell 110 into the second shell 120 (S100a), inserting the second shell 120 into the third shell 130 (S200a), filling each of the first shell 110, the second shell 120, and the third shell 130 with a filling material through the inlet 140 (S300a), and sealing the inlet 140 (S400a).

In step S100a, the first shell 110 may be inserted into the inner space of the second shell 120. In addition, after the first shell 110 is inserted into the inner space of the second shell 120, at least a portion of the first shell 110 may be combined with the second shell 120. Accordingly, the first shell 110 may be provided in a hanging state in the inner space of the second shell 120.

In step S200a, the second shell 120 including the first shell 110 may be inserted into the inner space of the third shell 130. In addition, after the second shell 120 is inserted into the inner space of the third shell 130, at least a portion of the second shell 120 may be combined with the third shell 130. Accordingly, the second shell 120 may be provided in a hanging state in the inner space of the third shell 130, and the first shell 110 may be provided in a hanging state in the inner space of the second shell 120.

In step S300a, the first shell 110, the second shell 120, and the third shell 130 may be filled with the first filling material Fm1, the second filling material Fm2, and the third filling material Fm3, respectively, through the inlet 140.

In an exemplary embodiment, the first shell 110 may be filled with the first filling material Fm1 through the first inlet 141, the second shell 120 may be filled with the second filling material Fm2 through the second inlet 143, and the third shell 130 may be filled with the third filling material Fm3 through the third inlet 145.

In step S400a, the inlets may be sealed to block leakage of the filling materials. In an exemplary embodiment, when the plurality of shells 110, 120, and 130 are filled with the filling materials Fm1, Fm2, and Fm3, respectively, through the plurality of first to third inlets 141, 143, and 145, the plurality of first to third inlets 141, 143, and 145 may be blocked by the sealing member described above.

In an exemplary embodiment, the sealing member may include an adhesive label that is easy to adhere to the plurality of shells 110, 120, and 130. For example, the sealing member may include a sticker-type adhesive label that is provided to have an area wider than the plurality of first to third inlets 141, 143, and 145 and blocks the first to third inlets 141, 143, and 145. However, the type of sealing member is not limited to that mentioned above.

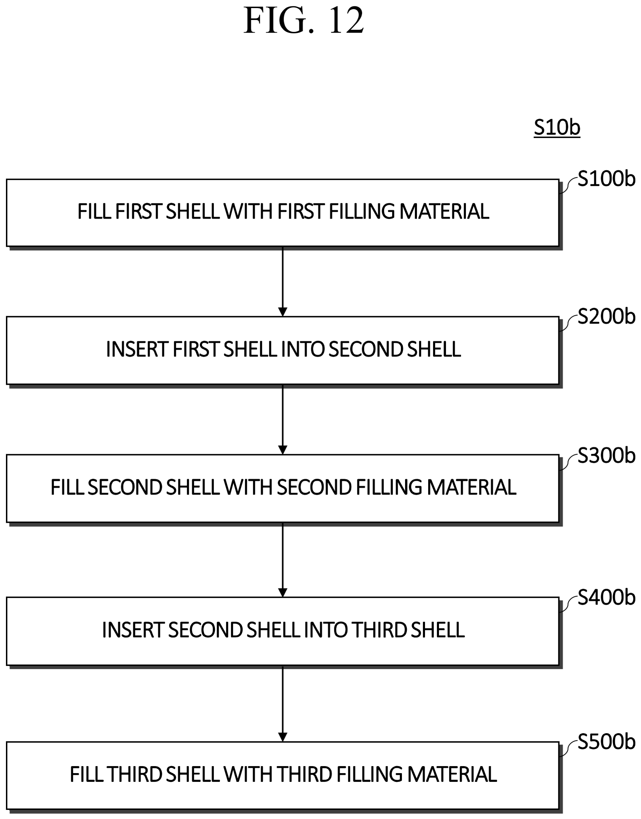

FIG. 12 is a flowchart showing a flow of a method S10b of manufacturing the breast implant 10 according to an exemplary embodiment of the present disclosure.

Referring to FIG. 12, the method S10b of manufacturing the breast implant 10 of the present disclosure may include filling the first shell 110 with the first filling material Fm1 (S100b), inserting the first shell 110 into the second shell 120 (S200b), filling the second shell 120 with the second filling material Fm2 (S300b), inserting the second shell 120 into the third shell 130 (S400b), and filling the third shell 130 with the third filling material (S500b).

In step S100b, the first shell 110 may be filled with the first filling material Fm1 through the first inlet 141. In addition, after the first shell 110 is filled with the first filling material Fm1, the first inlet 141 may be blocked using the sealing member.

In step S200b, the first shell 110 may be inserted into the inner space of the second shell 120. In addition, after the first shell 110 is inserted into the inner space of the second shell 120, at least a portion of the first shell 110 may be combined with the second shell 120. Accordingly, the first shell 110 may be provided in a hanging state in the inner space of the second shell 120.

In step S300b, the second shell 120 may be filled with the second filling material Fm2 through the second inlet 143. In addition, after the second shell 120 is filled with the second filling material Fm2, the second inlet 143 may be blocked using the sealing member.

In step S400b, the second shell 120 may be inserted into the inner space of the third shell 130. In addition, after the second shell 120 is inserted into the inner space of the third shell 130, at least a portion of the second shell 120 may be combined with the third shell 130. Accordingly, the second shell 120 may be provided in a hanging state in the inner space of the third shell 130, and the first shell 110 may be provided in a hanging state in the inner space of the second shell 120.

In step S500b, the third shell 130 may be filled with the third filling material Fm3 through the third inlet 145. In addition, after the third shell 130 is filled with the third filling material Fm3, the third inlet 145 may be blocked using the sealing member.

FIG. 13 is a view showing the breast implant 20 according to an exemplary embodiment of the present disclosure.

Referring to FIG. 13, the breast implant 20 of the present disclosure may include a first shell 210, a second shell 220, a first filling material Fm1a, a second filling material Fm2a, and an inlet 240.

The first shell 210 may be a shell accommodating the first filling material Fm1a. Specifically, the first shell 210 may be a shell providing an inner space in which the first filling material Fm1a is accommodated and may be a shell placed at an innermost side among the plurality of shells 210 and 220 included in the breast implant 20 of the present disclosure.

In an exemplary embodiment, a material of the first shell 210 may include silicone. However, the material of the first shell 210 is not limited to silicone and may also include another material. For example, the material of the first shell 210 may include a material such as flexible rubber. In addition, the material of the first shell 210 may include an organic compound or a polymer material having biocompatibility.

The first shell 210 may be placed inside the second shell 220 which will be described below. In an exemplary embodiment, the first shell 210 may be provided in an inner space of the second shell 220 in a state in which at least a portion of the first shell 210 is combined with the second shell 220. For example, at least a portion of the first shell 210 may be directly combined with the second shell 220, or at least a portion of the first shell 210 may be combined with the second shell 220 using a separate combining member.

In other words, at least a portion of the first shell 210 may be combined with the second shell 220, and the first shell 210 may be provided in a hanging state in the inner space of the second shell 220. Accordingly, the portion of the first shell 210 may freely move in the inner space of the second shell 220 based on an external force caused by movement of the body including the breast implant 20 of the present disclosure or based on gravity. For example, compared to a breast implant consisting of a single shell, movement may be easier for the plurality of shells included in the breast implant 20 of the present disclosure.

The first filling material Fm1a may be a material with which an inner space of the first shell 210 is filled. In an exemplary embodiment, a material of the first filling material Fm1a may include silicone. Specifically, the material of the first filling material Fm1a may be silicone provided in a gel form. In addition, the material of the first filling material Fm1a may be a saline solution. However, the material of the first filling material Fm1a is not limited to those mentioned above.

The first filling material Fm1a may have a first density. The density of the first filling material Fm1a may be defined as mass per unit volume of the first filling material Fm1a. For example, when the first filling material Fm1a includes a first silicone, a density of the first silicone may be defined as the first density.

In an exemplary embodiment, the first density of the first filling material Fm1a may be higher than a second density of the second filling material Fm2a with which the second shell 220, which will be described below, is filled.

In addition, the first filling material Fm1a may have a first viscosity. The viscosity of the first filling material Fm1a may be defined as a glutinous, sticky property of the first filling material Fm1a, and the viscosity of the first filling material Fm1a may be defined as the degree of viscosity of the first filling material Fm1a. For example, a high viscosity may mean relatively high resistance to a fluid flow, and a low viscosity may mean relatively low resistance to a fluid flow.

In an exemplary embodiment, the first viscosity of the first filling material Fm1a may be higher than a second viscosity of the second filling material Fm2a with which the second shell 220, which will be described below, is filled.

For example, a coefficient of viscosity of the first filling material Fm1a may be defined as a numerical value relating to viscosity of a liquid. For example, a first coefficient of viscosity of the first filling material Fm1a may be higher than a second coefficient of viscosity of the second filling material Fm2a with which the second shell 220, which will be described below, is filled.

In addition, the first filling material Fm1a may have a first cohesive force. The cohesive force of the first filling material Fm1a may be defined as an attractive force between molecules constituting the first filling material Fm1a. For example, when the first filling material Fm1a includes a first silicone, a cohesive force of the first silicone may be defined as the first cohesive force.

In an exemplary embodiment, the first cohesive force of the first filling material Fm1a may be greater than a second cohesive force of the second filling material Fm2a with which the second shell 220, which will be described below, is filled.

The breasts of the body are made up of mammary gland tissue and fat tissue surrounding the mammary gland tissue and thus tend to spread in the horizontal direction, which is perpendicular to the direction of gravity, due to gravity when a person is lying down and tend to drop in the vertical direction, which is parallel to the direction of gravity, due to gravity when the person is standing.

Since the first filling material Fm1a in the first shell 210 of the present disclosure can have the highest density, the highest viscosity, and the greatest cohesive force among the filling materials Fm1a and Fm2a with which the plurality of shells 210 and 220 are filled, the breast implant 20 of the present disclosure can implement movement similar to movement of the breasts of the body.

In addition, since the first filling material Fm1a in the first shell 210 of the present disclosure can have the highest density, the highest viscosity, and the greatest cohesive force among the filling materials Fm1a and Fm2a with which the plurality of shells 210 and 220 are filled, the breast implant 20 of the present disclosure can implement a texture similar to a texture of the breasts of the body.

The second shell 220 may be a shell accommodating the second filling material Fm2a. Specifically, the second shell 220 may be a shell providing an inner space in which the second filling material Fm2a is accommodated.

In an exemplary embodiment, a material of the second shell 220 may include silicone. However, the material of the second shell 220 is not limited to silicone and may also include another material. For example, the material of the second shell 220 may include a material such as flexible rubber. In addition, the material of the second shell 220 may include an organic compound or a polymer material having biocompatibility.

The breast implant 20 of the present disclosure may include the second shell 220 and the first shell 210 placed inside the second shell 220. Accordingly, even when the breast implant 20 of the present disclosure is inserted into the human body, and the second shell 220 placed at an outermost portion has relatively low mobility due to coming into contact with the body, relatively high mobility based on an external force caused by movement of the body or based on gravity can be secured for the first shell 210 placed inside the second shell 220. Accordingly, the breast implant 20 of the present disclosure can implement free movement of breasts according to movement of the body and gravity.

In addition, since the densities, viscosities, and cohesive forces of the filling materials Fm1a and Fm2a with which the plurality of shells 210 and 220 of the present disclosure are filled can gradually increase toward an inner side of the breast implant 20 (that is, gradually decrease toward an outer side of the breast implant 20), the breast implant 20 of the present disclosure can not only implement free movement of breasts according to movement of the body and gravity, but also implement a texture similar to the texture of the breasts.

In addition, due to differences in shapes and sizes of mammary gland tissue and fat tissue, the texture and shape of breasts may vary in patients who receive breast augmentation or breast reconstruction. The breast implant 20 of the present disclosure may include the plurality of shells 210 and 220, and the densities, viscosities, and cohesive forces of the plurality of filling materials Fm1a and Fm2a with which the plurality of shells 210 and 220 are filled may be individually determined according to states of breasts (for example, the shape, size, texture, and the like of the breasts) of each patient. In other words, materials of the plurality of filling materials Fm1a and Fm2a with which the plurality of shells 210 and 220 of the present disclosure are filled may be freely determined according to the states of breasts of each patient.

The second filling material Fm2a may be a material with which the inner space of the second shell 220 is filled. In an exemplary embodiment, a material of the second filling material Fm2a may include silicone. Specifically, the material of the second filling material Fm2a may be silicone provided in a gel form. In addition, the material of the second filling material Fm2a may be a saline solution. However, the material of the second filling material Fm2a is not limited to those mentioned above.

The second filling material Fm2a may have a second density. The density of the second filling material Fm2a may be defined as mass per unit volume of the second filling material Fm2a. For example, when the second filling material Fm2a includes a second silicone, a density of the second silicone may be defined as the second density.

**In** an exemplary embodiment, the second density of the second filling material Fm2a may be lower than the first density of the first filling material Fm1a with which the first shell 210 is filled. For example, when the first filling material Fm1a includes silicone having the first density, the second filling material Fm2a may include silicone having the second density lower than the first density.

However, the present disclosure is not limited thereto, and when the first filling material Fm1a includes silicone having the first density, the second filling material Fm2a may include a saline solution having the second density lower than the first density.

**In** addition, the second filling material Fm2a may have a second viscosity. The viscosity of the second filling material Fm2a may be defined as a glutinous, sticky property of the second filling material Fm2a, and the viscosity of the second filling material Fm2a may be defined as the degree of viscosity of the second filling material Fm2a.

**In** an exemplary embodiment, the second viscosity of the second filling material Fm2a may be lower than the first viscosity of the first filling material Fm1a with which the first shell 210 is filled.

The second filling material Fm2a may have a second cohesive force. The cohesive force of the second filling material Fm2a may be defined as an attractive force between molecules constituting the second filling material Fm2a. For example, when the second filling material Fm2a includes a second silicone, a cohesive force of the second silicone may be defined as the second cohesive force.

In an exemplary embodiment, the second cohesive force of the second filling material Fm2a may be smaller than the first cohesive force of the first filling material Fm1a with which the first shell 210 is filled.

The inlet 240 may provide a space through which the filling materials Fm1a and Fm2a are injected into the plurality of shells 210 and 220, respectively. In an exemplary embodiment, the inlet 240 may be provided at portions where the plurality of shells 210 and 220 are combined with each another (that is, come into contact with each another). For example, when the breast implant 20 is placed on a floor, the inlet 240 may be provided at an uppermost end of the breast implant 20 of the present disclosure.

The inlet 240 of the breast implant 20 of the present disclosure may include a first inlet providing a path through which the first filling material Fm1a is injected into the first shell 210 and a second inlet provided in a shape surrounding the first inlet and providing a path through which the second filling material Fm2a is injected into the second shell 220.

In addition, the breast implant 20 of the present disclosure may be provided in a shape similar to the shape of the breasts of the human body. At this time, the inlet 240 of the breast implant 20 may correspond to an areolar portion of the human body or a portion adjacent to the areolar portion. That is, when the breast implant 20 is inserted into the human body, the inlet 240 of the breast implant 20 may be provided at an areolar portion of the human body or a portion adjacent to the areolar portion.

An areolar portion has a relatively darker color than other portions of the body skin. After the breast implant 20 is inserted into the human body, control of injection or suction of the filling materials Fm1a and Fm2a inside the plurality of shells 210 and 220 may be necessary in some cases due to various causes such as discomfort and pain of a patient.

At this time, since the inlet 240 of the breast implant 20 can be present at an areolar portion of the human body or a portion adjacent to the areolar portion, even when an incision is made in a portion of the areolar portion or a portion adjacent to the areolar portion on the skin to control the filling materials Fm1a and Fm2a in the breast implant 20, visibility of a scar can be minimized due to a dark color of the areolar portion.

Since the breast implant 20 of the present disclosure includes the plurality of shells 210 and 220, and the breast implant 20 can be inserted into the body in a state in which only one shell 210 of the plurality of shells 210 and 220 is filled with the filling material Fm1a, the volume (that is, the size) of the breast implant 20 of the present disclosure at the time of inserting the breast implant 20 into the human body can be reduced. Accordingly, a range of an incision made in the skin for breast augmentation or breast reconstruction can be reduced, and the patient's satisfaction on the appearance can be enhanced.

For example, the breast implant 20 can be inserted into the human body in a state in which the first shell 210 is filled with the first filling material Fm1a including silicone. Accordingly, the volume of the breast implant 20 at the time of inserting the breast implant 20 into the human body can be reduced. Accordingly, a range of an incision made in the skin for breast augmentation or breast reconstruction can be reduced. In addition, the second shell 220 may be filled with the second filling material Fm2a including a saline solution after the breast implant 20 is inserted into the human body.

In addition, since the second shell 220 forming the exterior of the breast implant 20 of the present disclosure can be filled with a saline solution, even when the second shell 220 is damaged due to external impact and the second filling material Fm2a leaks, the second filling material Fm2a may be absorbed into the body, and thus the rate of capsular contracture can be reduced, and patients' mental stress about rupture of the breast implant 20 can be reduced.

The breast implant 20 of the present disclosure is not limited to the contents described with reference to FIG. 13 and may include all technical ideas described with reference to FIGS. 1 to 12.

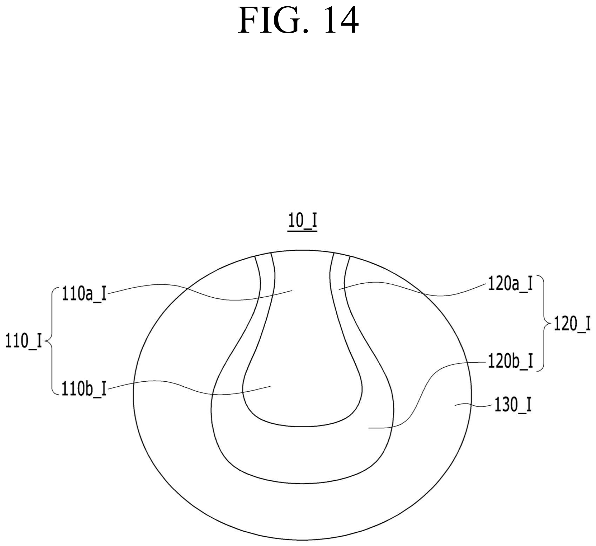

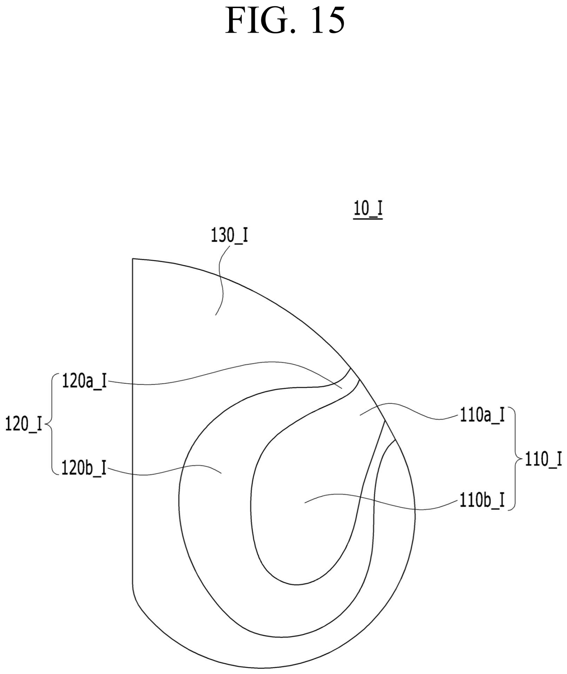

FIG. 14 is a view showing a structure of a breast implant 10_I according to an exemplary embodiment of the present disclosure. In addition, FIG. 15 is a view showing movements of a plurality of shells in the breast implant 10_I according to an exemplary embodiment of the present disclosure.

In describing the breast implant 10_I of FIGS. 14 and 15 below, differences from the breast implants 10 and 20 described above with reference to FIGS. 1 to 13 will be mainly described, and redundant description will be omitted.

Referring to FIGS. 14 and 15 together, the breast implant 10_I of the present disclosure may include a first shell 110_I, a second shell 120_I, and a third shell 130_I.

In an exemplary embodiment, the first shell 110_I may be provided in a shape similar to the shape of a water pocket in which an inlet portion through which a first filling material is injected droops (for example, the shape of a water balloon).

Specifically, the first shell 110_I may include a first hanging portion 110a_I and a first filling portion 110b_I. The first hanging portion 110a_I may be a portion of the first shell 110_I that is provided to provide the first filling portion 110b_I, which will be described below, in a hanging form inside the second shell 120_I. In addition, the first filling portion 110b_I may be a portion of the first shell 110_I that is provided to hang inside the second shell 120_I due to the first hanging portion 110a_I and has an inner space in which the first filling material is accommodated.

In an exemplary embodiment, the first hanging portion 110a_I may be provided to be combined with at least a portion of an inlet (not illustrated) or the second shell 120_I. In addition, the first filling portion 110b_I may be connected to and integrated with the first hanging portion 110a_I. For example, the first filling portion 110b_I and the first hanging portion 110a_I may include substantially the same materials.

In addition, in an exemplary embodiment, when the breast implant 10_I of the present disclosure is placed on a floor with the inlet facing upward, a horizontal cross-sectional area of the first hanging portion 110a_I of the first shell 110_I may be smaller than a horizontal cross-sectional area of the first filling portion 110b_I.

Since the first shell 110_I can include the first hanging portion 110a_I and the first filling portion 110b_I described above, the first filling portion 110b_I of the first shell 110_I of the present disclosure can be provided in a hanging form inside the second shell 120_I due to the first hanging portion 110a_I. Accordingly, mobility of the first filling portion 110b_I based on an external force or gravity can be improved.

The second shell 120_I may be provided in a shape similar to the shape of a water pocket in which an inlet portion through which a second filling material is injected droops (for example, the shape of a water balloon).

Specifically, the second shell 120_I may include a second hanging portion 120a_I and a second filling portion 120b_I. The second hanging portion 120a_I may be a portion of the second shell 120_I that is provided to provide the second filling portion 120b_I, which will be described below, in a hanging form inside the third shell 130_I. In addition, the second filling portion 120b_I may be a portion of the second shell 120_I that is provided to hang inside the third shell 130_I due to the second hanging portion 120a_I and has an inner space in which the second filling material is accommodated.

In an exemplary embodiment, the second hanging portion 120a_I may be provided to be combined with at least a portion of the inlet (not illustrated) or the third shell 130_I. In addition, the second filling portion 120b_I may be connected to and integrated with the second hanging portion 120a_I. For example, the second filling portion 120b_I and the second hanging portion 120a_I may include substantially the same materials.

In addition, in an exemplary embodiment, when the breast implant 10_I of the present disclosure is placed on a floor with the inlet facing upward, a horizontal cross-sectional area of the second hanging portion 120a_I of the second shell 120_I may be smaller than a horizontal cross-sectional area of the second filling portion 120b_I.

Since the second shell 120_I can include the second hanging portion 120a_I and the second filling portion 120b_I described above, the second filling portion 120b_I of the second shell 120_I of the present disclosure can be provided in a hanging form inside the third shell 130_I due to the second hanging portion 120a_I. Accordingly, mobility of the second filling portion 120b_I based on an external force or gravity can be improved.

That is, since the breast implant 10_I of the present disclosure can include the first shell 110_I including the first hanging portion 110a_I and the first filling portion 110b_1 and the second shell 120_I including the second hanging portion 120a_I and the second filling portion 120b_I, mobility of the first shell 110_I and the second shell 120_I based on an external force caused by movement of the body or based on gravity can be improved.

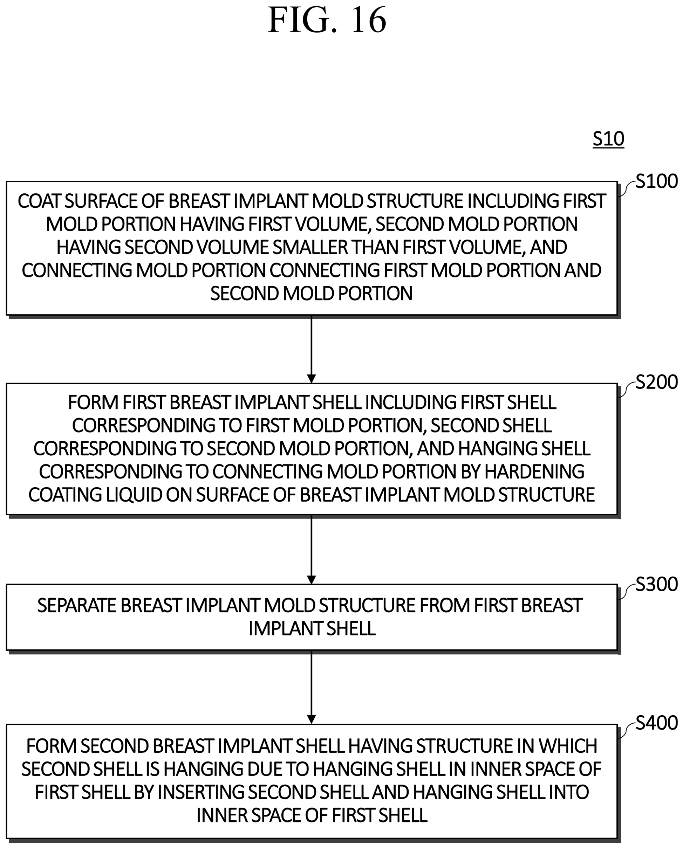

FIG. 16 is a flowchart showing a flow of a breast implant manufacturing method S10 according to an exemplary embodiment of the present disclosure.

The breast implant manufacturing method S10 of the present disclosure may be a method of manufacturing a breast implant including a plurality of shells. Specifically, the method of manufacturing a breast implant of the present disclosure may be a method of manufacturing a breast implant having a structure in which a second shell is inserted into a first shell and hanging inside the first shell.

Referring to FIG. 16, the breast implant manufacturing method S10 according to an exemplary embodiment of the present disclosure includes coating a surface of a breast implant mold structure including a first mold portion having a first volume, a second mold portion having a second volume smaller than the first volume, and a connecting mold portion connecting the first mold portion and the second mold portion (S100), forming a first breast implant shell including a first shell corresponding to the first mold portion, a second shell corresponding to the second mold portion, and a hanging shell corresponding to the connecting mold portion by hardening a coating liquid on the surface of the breast implant mold structure (S200), separating the breast implant mold structure from the first breast implant shell (S300), and forming a second breast implant shell having a structure in which the second shell is hanging due to the hanging shell in an inner space of the first shell by inserting the second shell and the hanging shell into the inner space of the first shell (S400).

Each step of the breast implant manufacturing method S10 of the present disclosure will be described in more detail below.

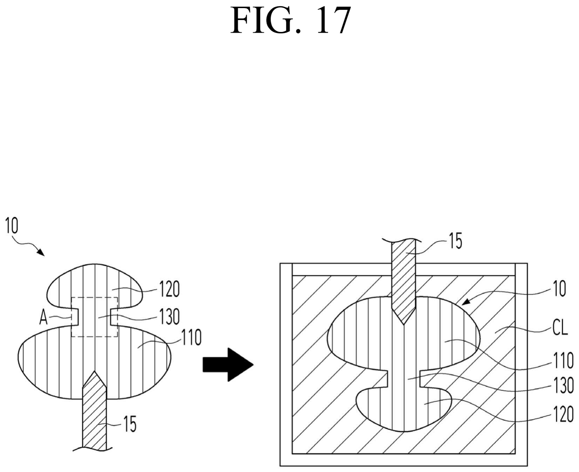

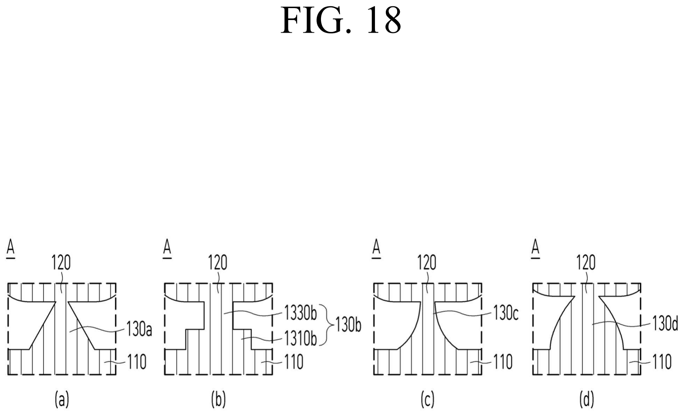

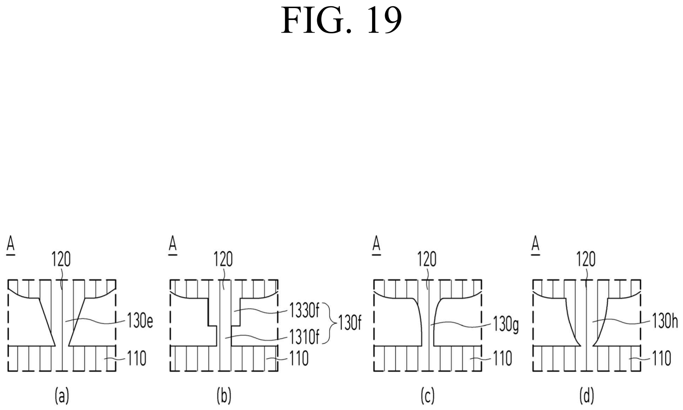

FIG. 17 is a view showing coating a surface of a breast implant mold structure 10 (S100) according to an exemplary embodiment of the present disclosure. In addition, FIGS. 18 and 19 are enlarged views of a connecting mold portion 130 of the breast implant mold structure 10 that is marked "A" in FIG. 17.

Referring to FIG. 17, in step S100, the surface of the breast implant mold structure 10 including a first mold portion 110, a second mold portion 120, and the connecting mold portion 130 may be coated with a coating liquid CL.

In an exemplary embodiment, a rod 15 may be combined with a lower portion of the breast implant mold structure 10 prior to performing step S100. In addition, step S100 may be a step of coating the surface of the breast implant mold structure 10 with the coating liquid CL by immersing the breast implant mold structure 10 in a water tank in which the coating liquid CL is accommodated.

However, the present disclosure is not limited thereto, and various other coating methods, such as a method of coating the surface of the breast implant mold structure 10 with the coating liquid CL by spraying the coating liquid CL onto the breast implant mold structure 10 using a spray, may be performed.

In an exemplary embodiment, the coating liquid CL may be a silicone fluid. However, the coating liquid CL is not limited to the silicone fluid and may include various other fluids. For example, the coating liquid may include a material such as flexible rubber. In addition, the material of the coating liquid may include an organic compound or a polymer material having biocompatibility.

In addition, the breast implant manufacturing method S10 of the present disclosure may include the coating of the surface of the breast implant mold structure 10 including the first mold portion 110 having a first volume, the second mold portion 120 having a second volume smaller than the first volume, and the connecting mold portion 130 connecting the first mold portion and the second mold portion (S100).

The breast implant mold structure 10 of the present disclosure may include the first mold portion 110 having a first volume, the second mold portion 120 having a second volume, and the connecting mold portion 130 connecting the first mold portion 110 and the second mold portion 120.

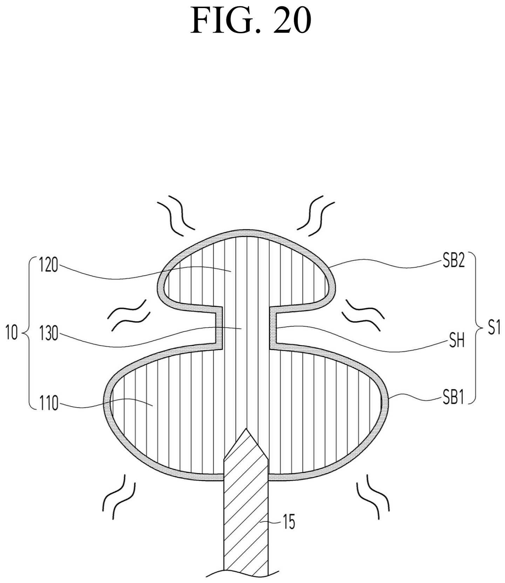

In an exemplary embodiment, the first mold portion 110 may be a mold provided to form a first shell SB1 (see FIG. 20) which will be described below. The first mold portion 110 may be provided in the shape of a breast. However, the present disclosure is not limited thereto, and the first mold portion 110 may be provided in other shapes such as a spherical shape or an elliptical spherical shape.

In an exemplary embodiment, the second mold portion 120 may be a mold provided to form a second shell SB2 (see FIG. 20) which will be described below. The second mold portion 120 may be provided to have a smaller volume than the first mold portion 110. Accordingly, the second shell SB2 may be formed to have a smaller volume than the first shell SB1, and the second shell SB2 can be easily inserted into an inner space of the first shell SB1 in step S400 which will be described below.

In an exemplary embodiment, the second mold portion 120 may be provided in the shape of a breast. However, the present disclosure is not limited thereto, and the second mold portion 120 may be provided in a spherical shape or an elliptical spherical shape.

In an exemplary embodiment, the second mold portion 120 may be provided in a shape whose length in the horizontal direction (that is, the horizontal direction in FIG. 17) gradually decreases away from the first mold portion 110. Accordingly, when the second shell SB2 formed through the second mold portion 120 is inserted into the inner space of the first shell SB1, the second shell SB2 may be provided in a shape that hangs and naturally droops in the first shell SB1. As a result, the breast implant including the first shell SB1 and the second shell SB2 can implement movement similar to movement of the breasts of the body.

In an exemplary embodiment, the second volume of the second mold portion 120 may be provided to range from about 25% to about 85% of the first volume of the first mold portion 110. In a case in which the second volume of the second mold portion 120 is less than 25% of the first volume of the first mold portion 110, when the second shell SB2 formed through the second mold portion 120 is inserted into the inner space of the first shell SB1 formed through the first mold portion 110, it is difficult to secure mobility of the second shell SB2 based on an external force caused by movement of the body or based on gravity. That is, when the second volume of the second mold portion 120 is less than 25% of the first volume of the first mold portion 110, since mobility of the second shell SB2 is not secured in the inner space of the first shell SB1, the breast implant including the first shell SB1 and the second shell SB2 is not able to implement natural movement of breasts.

In addition, in a case in which the second volume of the second mold portion 120 exceeds 85% of the first volume of the first mold portion 110, it may be difficult to perform the step of inserting the second shell SB2 formed through the second mold portion 120 into the inner space of the first shell SB1 formed through the first mold portion 110.

In addition, when the second volume of the second mold portion 120 exceeds 85% of the first volume of the first mold portion 110, excessively high mobility based on an external force caused by movement of the body or based on gravity may be secured for the second shell SB2 in the inner space of the first shell SB1, and physical damage may occur due to collision between the first shell SB1 and the second shell SB2. In addition, when the second volume of the second mold portion 120 exceeds 85% of the first volume of the first mold portion 110, the weight of the breast implant including the first shell SB1 and the second shell SB2 may be excessively heavy, and it may be difficult for a hanging shell SH to safely support the second shell SB2.

That is, since the second volume of the second mold portion 120 of the present disclosure can be provided to range from about 25% to about 85% of the first volume of the first mold portion 110, when the second shell SB2 formed through the second mold portion 120 is inserted into the inner space of the first shell SB1 formed through the first mold portion 110, relatively high mobility based on an external force caused by movement of the body or based on gravity may be secured for the second shell SB2, and the breast implant including the first shell SB1 and the second shell SB2 is able to implement natural movement of breasts.

In addition, since the second volume of the second mold portion 120 of the present disclosure can be provided to range from about 25% to about 85% of the first volume of the first mold portion 110, physical damage due to collision between the first shell SB1 and the second shell SB2 can be prevented, and the weight of the breast implant including the first shell SB1 and the second shell SB2 may be light.

The connecting mold portion 130 of the present disclosure will be described in more detail below with reference to FIGS. 18 and 19.

Referring to FIG. 18, the connecting mold portion 130 of the present disclosure may have a structure in which a length in the horizontal direction (that is, the horizontal direction in FIG. 18) gradually decreases away from the first mold portion 110 and toward the second mold portion 120.

Since the connecting mold portion 130 of the present disclosure illustrated in FIG. 18 can be provided to have the structure in which the length in the horizontal direction gradually decreases away from the first mold portion 110 and toward the second mold portion 120, the hanging shell SH (see FIG. 20) formed through the connecting mold portion 130 may be provided to have a structure in which a length in the horizontal direction gradually decreases away from a surface of the first shell SB1 in the inner space of the first shell SB1. Accordingly, the second shell SB2 may be provided to have a naturally hanging structure in the inner space of the first shell SB1 due to the hanging shell SH, and the second shell SB2 has relatively high mobility due to the shape of the hanging shell SH, and thus natural movement of breasts can be implemented.

In addition, since the connecting mold portion 130 of the present disclosure can be provided to have the structure in which the length in the horizontal direction gradually decreases away from the first mold portion 110 and toward the second mold portion 120, when the breast implant mold structure 10 is coated with the coating liquid CL while the breast implant mold structure 10 is placed upright with the second mold portion 120 facing upward, the coating liquid CL may flow downward along an inclined surface of the connecting mold portion 130 due to gravity, and the entire surface of the breast implant mold structure 10 can be uniformly coated with the coating liquid CL.

In an exemplary embodiment, as illustrated in (a) of FIG. 18, a connecting mold portion 130a may be provided to have a trapezoidal or triangular cross-section. Specifically, the connecting mold portion 130a may be provided in a tapered shape in which a length in the horizontal direction gradually decreases away from the first mold portion 110 and toward the second mold portion 120.

In an exemplary embodiment, as illustrated in (b) of FIG. 18, a connecting mold portion 130b may be provided to have a stepped, stair-shaped cross-section. Specifically, the connecting mold portion 130b may include a first stair region 1310b that is connected to the first mold portion 110 and has a first width in the horizontal direction and a second stair region 1330b that is interposed between the first stair region 1310b and the second mold portion 120 and has a second width smaller than the first width in the horizontal direction.

In an exemplary embodiment, as illustrated in (c) of FIG. 18, a connecting mold portion 130c may be provided to have a cross-section having a shape in which a length in the horizontal direction gradually decreases away from the first mold portion 110 and toward the second mold portion 120, and an outer surface of the connecting mold portion 130c may have a curved shape that is a shape recessed toward a central portion of the connecting mold portion 130c (for example, a concave pottery shape).

In an exemplary embodiment, as illustrated in (d) of FIG. 18, a connecting mold portion 130d may be provided to have a cross-section having a shape in which a length in the horizontal direction gradually decreases away from the first mold portion 110 and toward the second mold portion 120, and an outer surface of the connecting mold portion 130d may have a curved shape that is a shape protruding toward an outer region of the connecting mold portion (for example, a convex pottery shape).

In an exemplary embodiment, a length of the connecting mold portion 130d in a direction away from the first mold portion 110 and toward the second mold portion 120 (that is, the vertical direction) may range from about 0.2 cm to about 4.0 cm.

When the vertical length of the connecting mold portion 130d is less than 0.2 cm, the hanging shell SH formed through the connecting mold portion 130 may not be able to provide sufficient mobility to the second shell SB2 in the inner space of the first shell SB1. Accordingly, since mobility of the second shell SB2 is not secured in the inner space of the first shell SB1, the breast implant including the first shell SB1 and the second shell Sb2 is not able to implement natural movement of breasts.

In addition, when the vertical length of the connecting mold portion 130d exceeds 4.0 cm, the second shell SB2 may be provided in an excessively dropping shape in the inner space of the first shell SB1 due to the hanging shell SH. Accordingly, physical damage may occur due to collision between the second shell SB2 and the first shell SB1.

Since the vertical length of the connecting mold portion 130d of the present disclosure can range from about 0.2 cm to about 4.0 cm, when the second shell SB2 is inserted into the inner space of the first shell SB1, relatively high mobility based on an external force caused by movement of the body or based on gravity may be secured for the second shell SB2, and the breast implant including the first shell SB1 and the second shell SB2 is able to implement natural movement of breasts. In addition, physical damage due to collision between the first shell SB1 and the second shell SB2 can be prevented, and the weight of the breast implant including the first shell SB1 and the second shell SB2 may be light.

Referring to FIG. 19, the connecting mold portion 130 of the present disclosure may have a structure in which a length in the horizontal direction (that is, the horizontal direction in FIG. 19) gradually increases away from the first mold portion 110 and toward the second mold portion 120.

Since the connecting mold portion 130 of the present disclosure illustrated in FIG. 19 can be provided to have the structure in which the length in the horizontal direction gradually increases away from the first mold portion 110 and toward the second mold portion 120, when the breast implant mold structure 10 is coated with the coating liquid CL while the breast implant mold structure 10 is flipped upside down so that the second mold portion 120 faces downward (that is, the first mold portion 110 faces upward), the coating liquid CL may flow downward along an inclined surface of the connecting mold portion 130 due to gravity, and the entire surface of the breast implant mold structure 10 can be uniformly coated with the coating liquid CL.

In an exemplary embodiment, as illustrated in (a) of FIG. 19, a connecting mold portion 130e may be provided to have an inverted trapezoidal or inverted triangular cross-section. Specifically, the connecting mold portion 130e may be provided in a tapered shape in which a length in the horizontal direction gradually increases away from the first mold portion 110 and toward the second mold portion 120.

In an exemplary embodiment, as illustrated in (b) of FIG. 19, a connecting mold portion 130f may be provided to have a stepped, stair-shaped cross-section. Specifically, the connecting mold portion 130f may include a first stair region 1310f that is connected to the first mold portion 110 and has a first width in the horizontal direction and a second stair region 1330f that is interposed between the first stair region 1310f and the second mold portion 120 and has a second width larger than the first width in the horizontal direction. However, the connecting mold portion is not limited to the above and may further include two or more steps.

In an exemplary embodiment, as illustrated in (c) of FIG. 19, a connecting mold portion 130g may be provided to have a cross-section having a shape in which a length in the horizontal direction gradually increases away from the first mold portion 110 and toward the second mold portion 120, and an outer surface of the connecting mold portion 130g may have a curved shape that is a shape recessed toward a central portion of the connecting mold portion 130g (for example, a concave inverted pottery shape).

In an exemplary embodiment, as illustrated in (d) of FIG. 19, a connecting mold portion 130h may be provided to have a cross-section having a shape in which a length in the horizontal direction gradually increases away from the first mold portion 110 and toward the second mold portion 120, and an outer surface of the connecting mold portion 130h may have a curved shape that is a shape protruding toward an outer region of the connecting mold portion (for example, a convex inverted pottery shape).

In an exemplary embodiment, a length of the connecting mold portion 130h in a direction away from the first mold portion 110 and toward the second mold portion 120 (that is, the vertical direction) may range from about 0.2 cm to about 4.0 cm.

FIG. 20 is a view showing forming a first breast implant shell by hardening the coating liquid on the surface of the breast implant mold structure (S200) according to an exemplary embodiment of the present disclosure.

In an exemplary embodiment, step S200 of the present disclosure may be a step of forming a first breast implant shell S1 including the first shell SB1 corresponding to the first mold portion 110, the second shell SB2 corresponding to the second mold portion 120, and the hanging shell SH corresponding to the connecting mold portion 130 by hardening the coating liquid CL on the surface of the breast implant mold structure 10 (S200).

In an exemplary embodiment, in step S200, the coating liquid CL may be adhered to the breast implant mold structure 10 by hardening the coating liquid CL coated on the surface of the breast implant mold structure 10 of the present disclosure by natural drying or using a drying device. Accordingly, the first breast implant shell S1 including the first shell SB1 corresponding to the first mold portion 110, the second shell SB2 corresponding to the second mold portion 120, and the hanging shell SH corresponding to the connecting mold portion 130 may be formed.

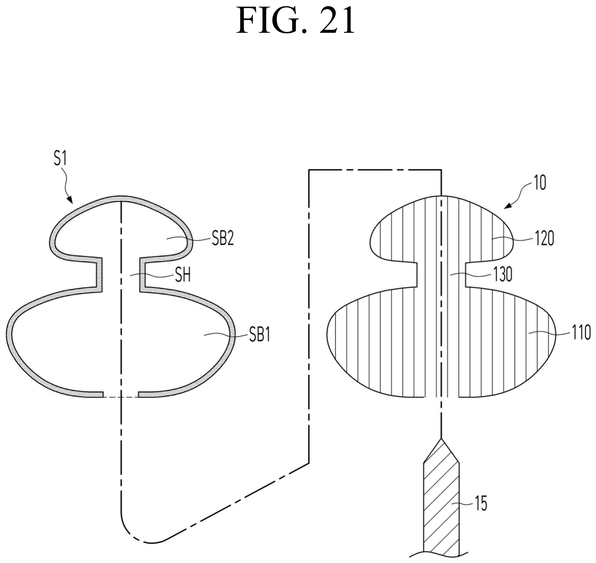

FIG. 21 is a view showing separating the breast implant mold structure 10 from the first breast implant shell S1 (S300) according to an exemplary embodiment of the present disclosure.

Referring to FIG. 21, in step S300, the rod 15 combined with the breast implant mold structure 10 may be removed. Then, an opening formed at a connecting portion between the breast implant mold structure 10 and the rod 15 may be widened to remove the first breast implant shell S1 from the breast implant mold structure 10 by flipping the first breast implant shell S1 upside down.

As the breast implant mold structure 10 is removed from the first breast implant shell S1, the first breast implant shell S1 of the present disclosure can have a structure including the first shell SB1 having a first inner space, the second shell SB2 having a second inner space, and the hanging shell SH connecting the first shell SB1 and the second shell SB2.

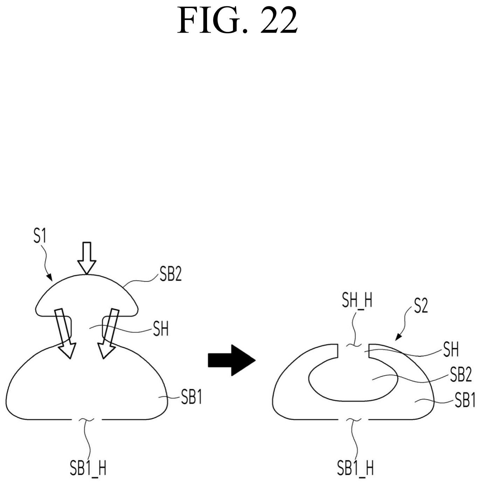

FIG. 22 is a view showing forming a second breast implant shell S2 by inserting the second shell SB2 and the hanging shell SH into the inner space of the first shell SB1 (S400) according to an exemplary embodiment of the present disclosure.

Referring to FIG. 22, in step S400, a step of inserting the second shell SB2 and the hanging shell SH into the inner space of the first shell SB1 may be performed. For example, step S400 may be performed by an operation of a machine (for example, a pushing machine) that applies an external force, which is in a direction toward the inner space of the first shell SH1, to the second shell SB2 and the hanging shell SH. However, a method of inserting the second shell SB2 and the hanging shell SH into the inner space of the first shell SB1 is not limited to the above.

That is, by performing step S400, the second shell SB2 and the hanging shell SH may be placed in the inner space of the first shell SB1, and as a result, the breast implant of the present disclosure may be formed to have a structure of the second breast implant shell S2 including a plurality of shells.

In an exemplary embodiment, as the second shell SB2 and the hanging shell SH are inserted into the inner space of the first shell SB1, an outer surface of the second shell SB2 and and an outer surface of the hanging shell SH of the first breast implant shell S1 illustrated in FIG. 21 may form an inner surface of the second shell SB2 and an inner surface of the hanging shell SH of the second breast implant shell S2 illustrated in FIG. 22, respectively.

In addition, in an exemplary embodiment, as the rod 15 is removed in step S300 described above, a first opening SB1_H may be formed at a lower portion of the second breast implant shell S2. In addition, in step S400, as the second shell SB2 and the hanging shell SH are inserted into the inner space of the first shell SB1, a second opening SH_H may be formed at an upper portion of the second breast implant shell S2.

Since steps S100 to S400 described above can be performed in the breast implant manufacturing method S10 according to an exemplary embodiment of the present disclosure, a breast implant including a plurality of shells can be manufactured by the breast implant manufacturing method S10 of the present disclosure. Accordingly, the breast implant manufactured using the manufacturing method of the present disclosure can implement a shape and a texture similar to those of the actual breasts made of mammary gland tissue and fat tissue and can implement natural movement of breasts.

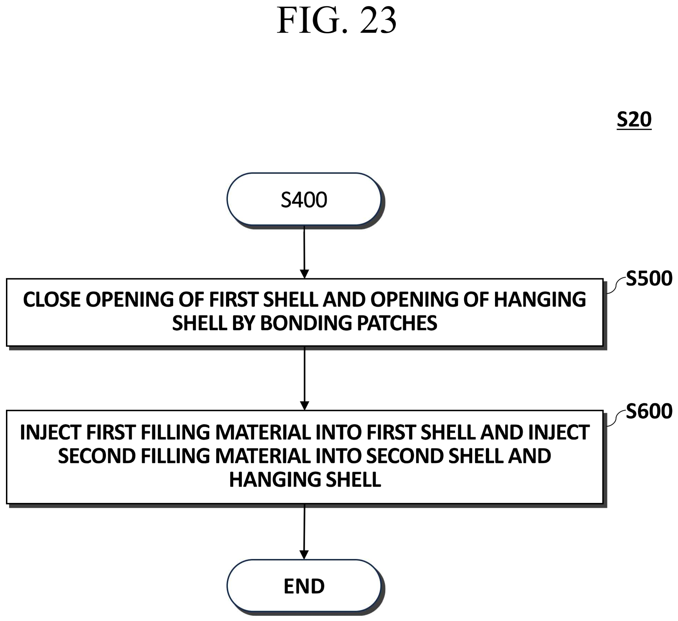

FIG. 23 is a flowchart showing a flow of a breast implant manufacturing method S20 according to an exemplary embodiment of the present disclosure.

The breast implant manufacturing method S20 of the present disclosure may be performed after the breast implant manufacturing method S10 described above with reference to FIGS. 16 to 22. In describing the breast implant manufacturing method S20 below, differences from the breast implant manufacturing method S10 described above with reference to FIGS. 16 to 22 will be mainly described, and redundant description will be omitted.

The breast implant manufacturing method S20 of the present disclosure may include closing the first opening SB1_H of the first shell SB1 and the second opening SH_H of the hanging shell SH by bonding patches 310 and 330 (S500) and injecting a first filling material into the first shell SB1 and injecting a second filling material into the second shell SB2 and the hanging shell SH (S600). Steps S500 and S600 of the present disclosure may be performed after step S400 described above with reference to FIG. 16.

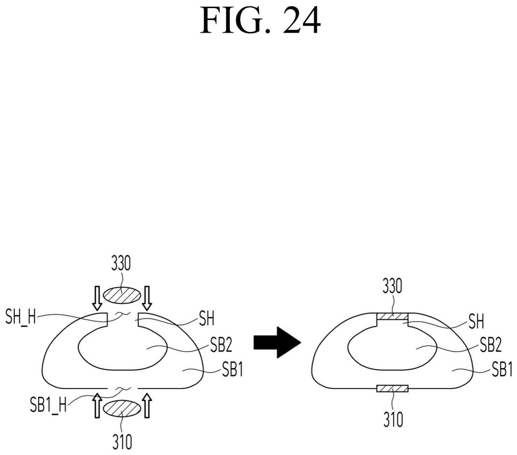

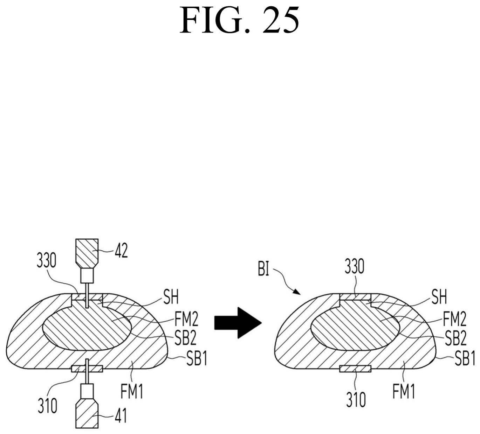

The breast implant manufacturing method S20 of the present disclosure will be described in detail below with reference to FIGS. 24 and 25.

FIG. 24 is a view showing the closing of the first opening SB1_H of the first shell SB1 and the second opening SH_H of the hanging shell SH by bonding the patches 310 and 330 (S500) of the present disclosure.

Referring to FIG. 24, in step S500, a first patch 310 may be attached to the first opening SB1_H of the first shell SB1, and the first opening SB1_H may be closed. In an exemplary embodiment, the first patch 310 may close the first opening SB1_H by being attached to an inner surface of the first shell SB1 adjacent to the first opening SB1_H after being inserted into the first opening SB1_H.

In addition, in step S500, a second patch 330 may be attached to the second opening SH_H of the hanging shell SH, and the second opening SH_H may be closed. In an exemplary embodiment, the second patch 330 may close the second opening SH_H by being attached to an inner surface of the hanging shell SH adjacent to the second opening SH_H after being inserted into the second opening SH_H.

In addition, the first patch 310 and the second patch 330 may each have elasticity and physical properties substantially the same as those of the first shell SB1, the second shell SB2, and the hanging shell SH. In an exemplary embodiment, materials of the first patch 310 and the second patch 330 may include the same materials as the first shell SB1, the second shell SB2, and the hanging shell SH. For example, the materials of the first patch 310 and the second patch 330 may include silicone.

FIG. 25 is a view showing injecting a first filling material FM1 into the first shell SB1 and injecting a second filling material FM2 into the second shell SB2 and the hanging shell SH (S600) of the present disclosure.

Referring to FIG. 25, in step S600, the first filling material FM1 may be injected into the inner space of the first shell SB1 using a syringe, and the second filling material FM2 may be injected into the inner spaces of the second shell SB2 and the hanging shell SH using a syringe.

In an exemplary embodiment, the first filling material FM1 and the second filling material FM2 may include a saline solution, a silicone gel, and a hydrogel. However, the types of the first filling material FM1 and the second filling material FM2 are not limited to the above.

In an exemplary embodiment, in step S600, a syringe may be inserted into the first patch 310, and the first filling material FM1 may be injected into the inner space of the first shell SB1. In addition, in step S600, a syringe may be inserted into the second patch 330, and the second filling material FM2 may be injected into the inner space of the second shell SB2.

At this time, the first filling material FM1 may have a first density. The density of the first filling material FM1 may be defined as mass per unit volume of the first filling material FM1. For example, when the first filling material FM1 includes a first silicone, a density of the first silicone may be defined as the first density.

In an exemplary embodiment, the first density of the first filling material FM1 with which the first shell SB1 is filled may be lower than a second density of the second filling material FM2 with which the second shell SB2 is filled.

In addition, the first filling material FM1 may have a first viscosity. The viscosity of the first filling material FM1 may be defined as a glutinous, sticky property of the first filling material FM1, and the viscosity of the first filling material FM1 may be defined as the degree of viscosity of the first filling material FM1. For example, a high viscosity may mean relatively high resistance to a fluid flow, and a low viscosity may mean relatively low resistance to a fluid flow.

In an exemplary embodiment, the first viscosity of the first filling material FM1 with which the first shell SB1 is filled may be lower than a second viscosity of the second filling material FM2 with which the second shell SB2 is filled.

For example, a coefficient of viscosity of the first filling material FM1 may be defined as a numerical value relating to viscosity of a liquid. For example, a first coefficient of viscosity of the first filling material FM1 may be lower than a second coefficient of viscosity of the second filling material FM2 with which the second shell SB2 is filled.

In addition, the first filling material FM1 with which the first shell SB1 is filled may have a first cohesive force. The cohesive force of the first filling material FM1 may be defined as an attractive force between molecules constituting the first filling material FM1. For example, when the first filling material FM1 includes a first silicone, a cohesive force of the first silicone may be defined as the first cohesive force.

In an exemplary embodiment, the first cohesive force of the first filling material FM1 may be smaller than a second cohesive force of the second filling material FM2 with which the second shell SB2 is filled.

The breasts of the body are made up of mammary gland tissue and fat tissue surrounding the mammary gland tissue and thus tend to spread in the horizontal direction, which is perpendicular to the direction of gravity, due to gravity when a person is lying down and tend to drop in the vertical direction, which is parallel to the direction of gravity, due to gravity when the person is standing.

Since the breast implant manufactured using the breast implant manufacturing method S20 of the present disclosure includes a plurality of shells, and a filling material with which an inner shell is filled can have a relatively higher density, a relatively higher viscosity, and a relatively greater cohesive force than a filling material with which an outer shell is filled, the breast implant manufactured using the breast implant manufacturing method of the present disclosure can implement movement similar to movement of the breasts of the body.

In addition, since the breast implant manufactured using the breast implant manufacturing method S20 of the present disclosure includes a plurality of shells, and a filling material with which an inner shell is filled can have a relatively higher density, a relatively higher viscosity, and a relatively greater cohesive force than a filling material with which an outer shell is filled, the breast implant manufactured using the breast implant manufacturing method of the present disclosure can implement a texture similar to a texture of the breasts of the body.

In an exemplary embodiment, the first filling material FM1 with which the first shell SB1 is filled may be a saline solution, and the second filling material FM2 with which the second shell SB2 is filled may be silicone. Since the breast implant manufactured using the breast implant manufacturing method of the present disclosure includes a plurality of shells, and an outermost shell of the plurality of shells can include a saline solution as a filling material, when the breast implant is inserted into the body, the size of an incision scar in a patient can be reduced, and the patient's satisfaction on the appearance can be enhanced.

In an exemplary embodiment, the first filling material FM1 with which the first shell SB1 is filled may be water, and the second filling material FM2 with which the second shell SB2 is filled may be silicone having a higher density than water. For example, the density of silicone may range from about 1.01 g/ml to about 1.20 g/ml and may have a higher value than the density of water. Accordingly, since the breast implant manufactured using the breast implant manufacturing method of the present disclosure includes a plurality of shells, and an outermost shell of the plurality of shells can include water having a lower density than silicone as a filling material, when the breast implant is inserted into the body, the size of an incision scar in a patient can be reduced, and the patient's satisfaction on the appearance can be enhanced.

In addition, conventionally, whether rupture of a breast implant has occurred is determined through detailed ultrasound imaging or magnetic resonance imaging (MRI). Consequently, the time for removing or replacing a breast implant is delayed, or an inflammatory response increases, causing various complications including capsular contracture. Since the outer shell forming the exterior of the breast implant manufactured using the breast implant manufacturing method of the present disclosure can include a saline solution, even when the outer shell is damaged due to external impact and the filling material leaks, from the size of the breast implant getting smaller, the patient can immediately recognize that rupture of the breast implant has occurred, and the physiological saline solution is safely absorbed into the human body. As a result, the patient can immediately plan to receive surgery for removing or replacing the breast implant, and capsular contractures and inflammatory responses that occur when the breast implant is left in a ruptured state for a long period of time can be reduced. That is, patients' mental stress about rupture of breast implants can be reduced.

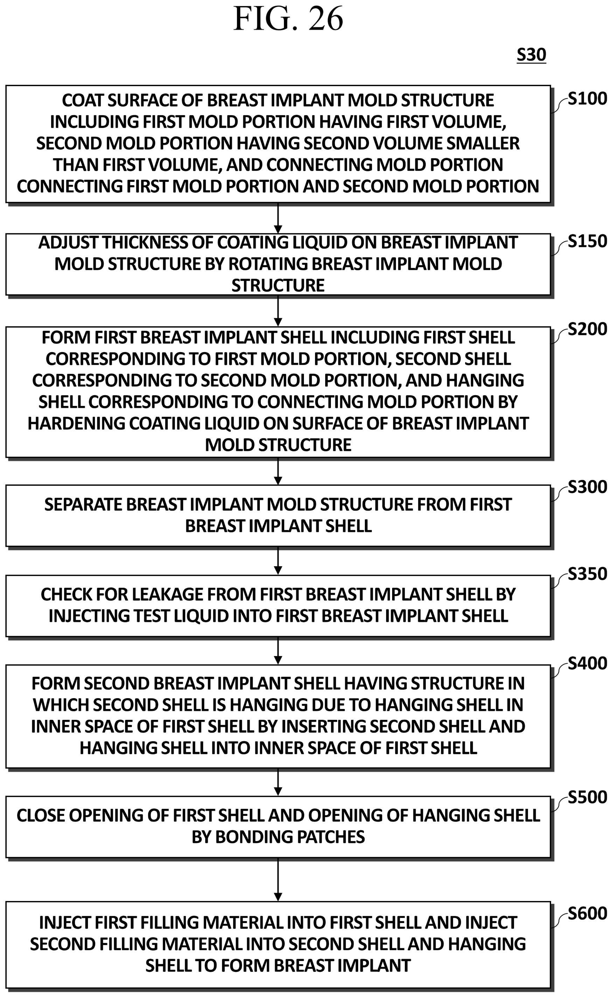

FIG. 26 is a flowchart showing a flow of a breast implant manufacturing method S30 according to an exemplary embodiment of the present disclosure.

Referring to FIG. 26, the breast implant manufacturing method S30 of the present disclosure may include coating a surface of a breast implant mold structure including a first mold portion having a first volume, a second mold portion having a second volume smaller than the first volume, and a connecting mold portion connecting the first mold portion and the second mold portion (S100), adjusting a thickness of a coating liquid on the breast implant mold structure by rotating the breast implant mold structure (S150), forming a first breast implant shell including a first shell corresponding to the first mold portion, a second shell corresponding to the second mold portion, and a hanging shell corresponding to the connecting mold portion by hardening the coating liquid on the surface of the breast implant mold structure (S200), separating the breast implant mold structure from the first breast implant shell (S300), checking for leakage from the first breast implant shell by injecting a test liquid into the first breast implant shell (S350), forming a second breast implant shell having a structure in which the second shell is hanging due to the hanging shell in an inner space of the first shell by inserting the second shell and the hanging shell into the inner space of the first shell (S400), closing an opening of the first shell and an opening of the hanging shell by bonding patches (S500), and injecting a first filling material into the first shell and injecting a second filling material into the second shell and the hanging shell to form a breast implant (S600).

In describing the breast implant manufacturing method S30 of the present disclosure below, differences from the breast implant manufacturing methods S10 and S20 of the present disclosure described above with reference to FIGS. 16 to 25 will be mainly described, and redundant description will be omitted.

The breast implant manufacturing method S30 of the present disclosure may further include adjusting a thickness of a coating liquid on the breast implant mold structure by rotating the breast implant mold structure (S150).

When a thickness is formed to be the same across all parts of a breast implant, in a case in which the breast implant is inserted into the body, due to a filling material leaning downward, a rippling phenomenon in which a portion of the breast implant is folded may occur. When the rippling phenomenon repeatedly occurs at a specific part, there is a risk of rupture of the breast implant due to weakening of physical properties of the corresponding part.

To address this issue, the breast implant manufacturing method S30 of the present disclosure may further include rotating the breast implant mold structure 10 on which the coating liquid L is applied (S150). By performing step S150, a side portion of the first breast implant shell S1 can be strengthened.

In an exemplary embodiment, in step S150, the breast implant mold structure 10 may be rotated so that a peak end of the breast implant mold structure 10 faces upward. In addition, in step S150, the breast implant mold structure 10 may be rotated so that the peak end of the breast implant mold structure 10 faces downward. For example, step S150 may be performed by alternately performing rotating the breast implant mold structure 10 so that the peak end of the breast implant mold structure 10 faces upward and rotating the breast implant mold structure 10 so that the peak end of the breast implant mold structure 10 faces downward.

Specifically, when the breast implant mold structure 10 is rotated so that the peak end of the breast implant mold structure 10 faces upward, since the coating liquid CL moves to a side portion of the breast implant mold structure 10 due to gravity and a centrifugal force, a thickness of a portion adjacent to a peak end of the first breast implant shell S1 may be formed relatively thin. Conversely, when the breast implant mold structure 10 is rotated so that the peak end of the breast implant mold structure 10 faces downward, since the coating liquid CL moves to the peak end of the breast implant mold structure 10 due to gravity, a thickness of a portion adjacent to the peak end may be formed relatively thick.

In this way, by alternately performing the rotating of the breast implant mold structure 10 so that the peak end of the breast implant mold structure 10 faces upward and the rotating of the breast implant mold structure 10 so that the peak end of the breast implant mold structure 10 faces downward, the thickness of the first breast implant shell S1 can be easily adjusted in the method of the present disclosure.

By performing step S150, the size of a thickness of a portion where rippling frequently occurs can be controlled, and as a result, folding resistance of the portion may increase, and durability of the portion may increase.

The breast implant manufacturing method S30 of the present disclosure may include checking for leakage from the first breast implant shell by injecting a test liquid into the first breast implant shell (S350). The test liquid may include water, but the type of test liquid is not limited thereto.

In an exemplary embodiment, in step S350, a test liquid may be injected into the first breast implant shell S1 separated from the breast implant mold structure 10. For example, in step S350, a test liquid having a volume greater than a volume of the first breast implant shell S1 may be injected into an inner space of the first breast implant shell S1 to inflate the first breast implant shell S1.

At this time, when the volume of the first breast implant shell S1 increases compared to the original volume due to elasticity of the first breast implant shell S1 itself after the test liquid is injected into the first breast implant shell S1, it may be determined that there is no leakage from the first breast implant shell S1.

In addition, when the volume of the first breast implant shell S1 does not increase compared to the original volume, or the test liquid is discharged from a portion of the first breast implant shell S1 after the test liquid is injected into the first breast implant shell S1, it may be determined that there is leakage from the first breast implant shell S1.

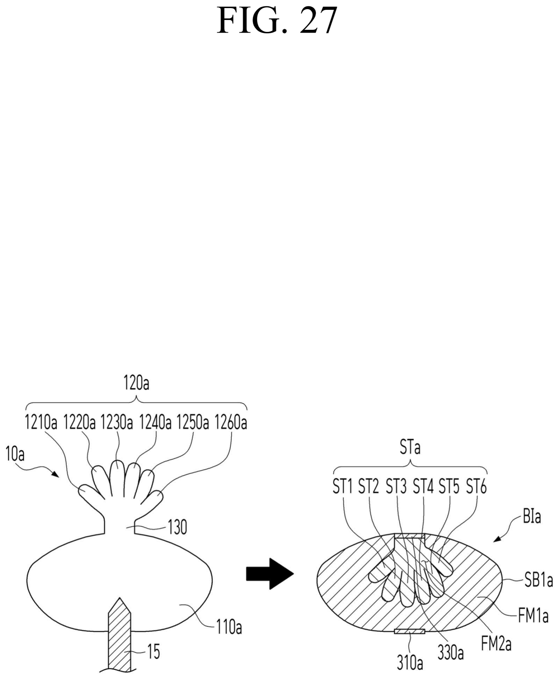

FIG. 27 is a view showing coating a surface of a breast implant mold structure 10a according to an exemplary embodiment of the present disclosure.

Referring to FIG. 27, the breast implant mold structure 10a may include a first mold portion 110a, a second mold portion 120a, and a connecting mold portion 130.

In an exemplary embodiment, the second mold portion 120a may include a plurality of shell pockets 1210a to 1260a. As illustrated in FIG. 27, the plurality of shell pockets 1210a to 1260a my be formed to protrude outward from a central portion and may form a bundle structure. For example, the plurality of shell pockets 1210a to 1260a may each protrude outward from the central portion, and the second mold portion 120a may be provided in the shape of mammary gland tissue or the shape of a palm.

In addition, in an exemplary embodiment, the plurality of shell pockets may each include a bar-shaped shell and an elliptical shell that extends from the bar-shaped shell and has a greater volume (or a larger width) than the bar-shaped shell. Accordingly, the breast implant manufactured using the breast implant mold structure of the present disclosure can better implement the shape of mammary gland tissue.

In addition, in an exemplary embodiment, the plurality of shell pockets may have a structure having a plurality of through-holes that pass through the plurality of shell pockets. That is, when the plurality of shell pockets are viewed in a plan view, the plurality of shell pockets may have a plurality of holes that pass through surfaces of the plurality of shell pockets. Accordingly, due to the plurality of shell pockets having the through-holes, the weight of the breast implant manufactured using the breast implant mold structure of the present disclosure can be redued.

In an exemplary embodiment, when a second shell STa that corresponds to the second mold portion 120a is inserted into an inner space of a first shell SB1a, the shape of the second shell STa may be provided to be similar to the shape of mammary gland tissue of a breast. Accordingly, when the second shell STa is filled with a second filling material FM2a, a breast implant BIa including the first shell SB1a and the second shell STa may be provided in a shape similar to the shape of the breast of the body and can implement a texture and movement similar to those of the breast of the body.

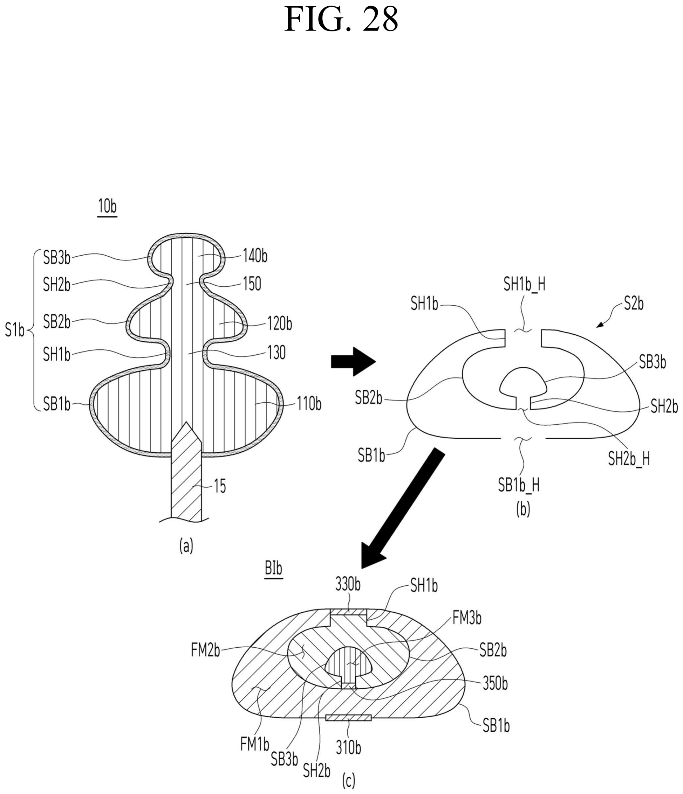

FIG. 28 is a view showing a method of manufacturing a breast implant BIb using a breast implant mold structure 10b according to an exemplary embodiment of the present disclosure.

In describing the breast implant mold structure 10b of the present disclosure below, differences from the breast implant mold structure 10 of FIG. 16 will be mainly described, and redundant description will be omitted.

Referring to FIG. 28, the breast implant mold structure 10b of the present disclosure may include a first mold portion 110b, a second mold portion 120b, a first connecting mold portion 130, a third mold portion 140b, and a second connecting mold portion 150.

At this time, the first connecting mold portion 130 may be a portion of the breast implant mold structure 10b that connects the first mold portion 110b and the second mold portion 120b. In addition, the second connecting mold portion 150 may be a portion of the breast implant mold structure 10b that connects the second mold portion 120b and the third mold portion 140b.

In an exemplary embodiment, a first volume of the first mold portion 110b may be provided to be greater than a second volume of the second mold portion 120b. In addition, the second volume of the second mold portion 120b may be provided to be greater than a third volume of the third mold portion 140b.

Accordingly, a volume of a first shell SB1b that corresponds to the first mold portion 110b may be provided to be greater than a volume of a second shell SB2b that corresponds to the second mold portion 120b. In addition, the volume of the second shell SB2b that corresponds to the second mold portion 120b may be provided to be greater than a volume of a third shell SB3b that corresponds to the third mold portion 140b.

In an exemplary embodiment, the second shell SB2b may be accommodated in an inner space of the first shell SB1b, and the second shell SB2b may be provided in a hanging state in the first shell SB1b due to a first hanging shell SH1b. In addition, the third shell SB3b may be accommodated in an inner space of the second shell SB2b, and the third shell SB3b may be provided in a hanging state in the second shell SB2b due to a second hanging shell SH2b.

In an exemplary embodiment, a second breast implant shell S2b of the present disclosure may be manufactured as the third shell SB3b is inserted into the second shell SB2b and the second shell SB2b is inserted into the first shell SB1b.

At this time, the second breast implant shell S2b may have a first opening SB1b_H formed in the first shell SB1b due to removal of a rod, a second opening SH1b_H formed due to insertion of the second shell SB2b and the first hanging shell SH1b into the first shell SB1b, and a third opening SH2b_H formed due to insertion of the third shell SB3b and the second hanging shell SH2b into the second shell SB2b.

For example, the first opening SB1b_H and the third opening SH2b_H may be provided at a lower portion of the second breast implant shell S2b, and the second opening SH1b_H may be provided at an upper portion of the second breast implant shell S2b.

In an exemplary embodiment, the first opening SB1b_H, the second opening SH1b_H, and the third opening SH2b_H may be closed by a first patch 310b, a second patch 330b, and a third patch 350b, respectively. Since the technical idea of closing an opening through a patch is the same as described above, detailed description thereof will be omitted.

In an exemplary embodiment, the third opening SH2b_H may be closed through the third patch 350b first before the first opening SB1b_H is closed through the first patch 310b. That is, the first opening SB1b_H may be closed through the first patch 310b after the third opening SH2b_H is closed through the third patch 350b.

Then, the first shell SB1b, the second shell SB2b, and the third shell SB3b may each filled with a filling material. Sine the technical idea of filling each of a plurality of shells with a filling material is the same as described above, detailed description thereof will be omitted.

Since the breast implant BIb manufactured using the breast implant manufacturing method of the present disclosure includes a plurality of shells, and a filling material with which an inner shell is filled can have a relatively higher density, a relatively higher viscosity, and a relatively greater cohesive force than a filling material with which an outer shell is filled, the breast implant manufactured using the breast implant manufacturing method of the present disclosure can implement movement similar to movement of the breasts of the body.

In addition, since the breast implant manufactured using the breast implant manufacturing method of the present disclosure includes a plurality of shells, and a filling material with which an inner shell is filled can have a relatively higher density, a relatively higher viscosity, and a relatively greater cohesive force than a filling material with which an outer shell is filled, the breast implant manufactured using the breast implant manufacturing method of the present disclosure can implement a texture similar to a texture of the breasts of the body.

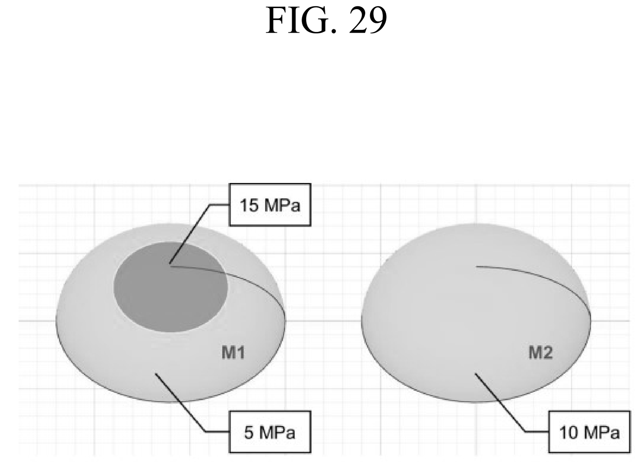

FIG. 29 is a view showing a breast implant according to a comparative example and a breast implant of the present disclosure.

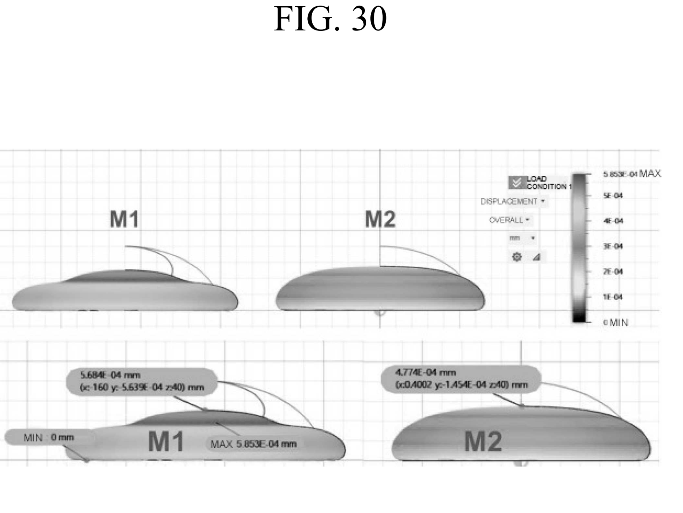

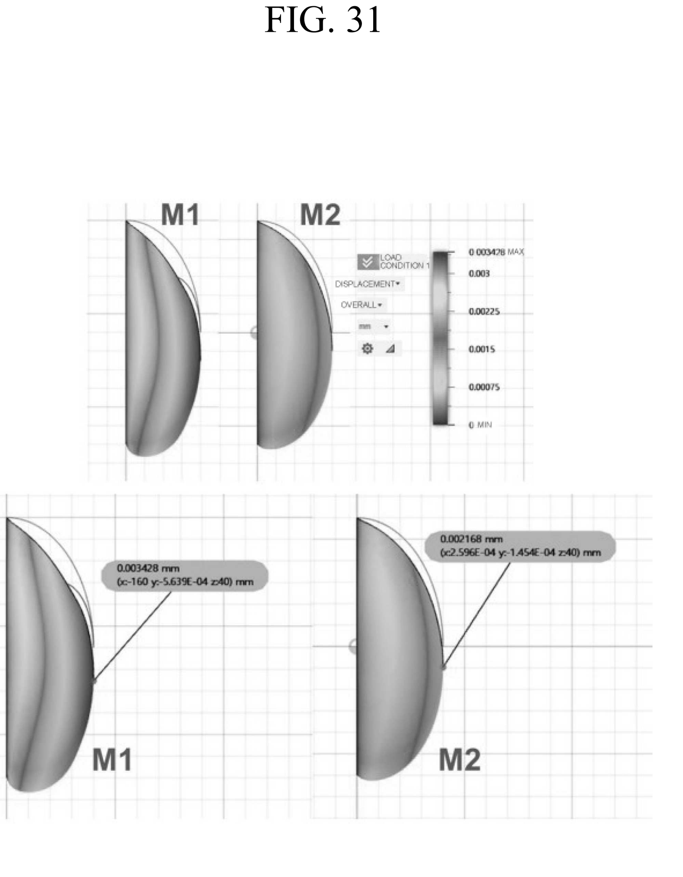

In addition, FIG. 30 is a view showing shapes of the breast implant according to the comparative example and the breast implant of the present disclosure when they are placed on a flat surface. and FIG. 31 is a view showing shapes of the breast implant according to the comparative example and the breast implant of the present disclosure when they are placed on a vertical surface.

Referring to FIG. 29, a breast implant M1 of the present disclosure may be a breast implant including a first shell (outer shell) and a second shell (inner shell). Since the technical ideas of the breast implant and a method of manufacturing the same of the present disclosure are the same as those described above with reference to FIGS. 16 to 28, detailed description thereof will be omitted. In addition, a breast implant M2 according to a comparative example may be a breast implant consisting of a single shell.

In one embodiment, a first elastic modulus of the first shell may be lower than a second elastic modulus of the second shell. The elastic modulus may indicate a degree of resistance of a material to a tensile force or a compressive force. For example, silicone may have an elastic modulus that ranges from 3 MPA to 15 MPA.

In one embodiment, a first elastic modulus of a first filling material with which the first shell (outer shell) is filled may be lower than a second elastic modulus of a second filling material with which the second shell (inner shell) is filled. For example, the first elastic modulus of the first shell may range from about 1 MPA to 7 MPA, and the second elastic modulus of the second shell may range from 8 MPA to 17 MPA.

Referring to FIG. 29, in the breast implant M1 of the present disclosure, the outer shell may have the first elastic modulus of 5 MPA, and the inner shell may have the second elastic modulus of 15 MPA. In addition, the breast implant M2 according to the comparative example that is illustrated in FIG. 29 may be a breast implant made of a single shell that has an elastic modulus of 10 MPA.

Prior to giving description with reference to FIGS. 30 and 31, note that a horizontal length (that is, width) of each of the breast implant of the present disclosure and the breast implant according to the comparative example that are used for simulation of FIGS. 30 and 31 may be about 12 cm. In addition, a vertical length (that is, height) of each of the breast implant of the present disclosure and the breast implant according to the comparative example that are used for simulation may be about 4 cm. In addition, a horizontal length of the second shell (that is, the inner shell) of the breast implant of the present disclosure may be about 6 cm, and a vertical length thereof may be about 3 cm.

Referring to FIG. 30, when the breast implant consisting of a plurality of shells of the present disclosure and the breast implant consisting of a single shell according to the comparative example were placed on a flat surface (that is, a floor surface), displacement of 5.68×10⁻⁴ mm occurred in the breast implant of the present disclosure, and displacement of 4.77×10⁻⁴ mm occurred in the breast implant according to the comparative example.

That is, strain of the height of the breast implant of the present disclosure is higher than strain of the height of the breast implant according to the comparative example. Accordingly, a change in the width (that is, a change in the horizontal length) of the breast implant of the present disclosure may be greater than a change in the width of the breast implant according to the comparative example.

Accordingly, compared to the breast implant according to the comparative example, the breast implant of the present disclosure can better implement movement of breasts when a person is lying down.

That is, since the breast implant manufactured using the breast implant manufacturing method of the present disclosure includes a plurality of shells, and a filling material with which an inner shell is filled can have a relatively higher elastic modulus than a filling material with which an outer shell is filled, the breast implant manufactured using the breast implant manufacturing method of the present disclosure can implement movement similar to movement of breasts when a person is lying down.

Referring to FIG. 31, when the breast implant consisting of a plurality of shells of the present disclosure and the breast implant consisting of a single shell according to the comparative example were placed on a vertical surface, displacement of 3.43×10⁻⁴ mm occurred in the direction of gravity in the breast implant of the present disclosure, and displacement of 2.17×10⁻⁴ mm occurred in the breast implant according to the comparative example. That is, the breast implant of the present disclosure can better implement dropping of breasts due to gravity.

In addition, when the breast implant consisting of a plurality of shells of the present disclosure and the breast implant consisting of a single shell according to the comparative example are placed on a vertical surface, as illustrated in FIG. 31, the breast implant of the present disclosure can implement a shape similar to a shape in which breasts drop due to gravity.

That is, since the breast implant manufactured using the breast implant manufacturing method of the present disclosure includes a plurality of shells, and a filling material with which an inner shell is filled can have a relatively higher elastic modulus than a filling material with which an outer shell is filled, the breast implant manufactured using the breast implant manufacturing method of the present disclosure can implement movement similar to movement of breasts when a person is standing.

As described above, exemplary embodiments have been disclosed in the drawings and the specification. Although specific terms have been used to describe the embodiments in the present specification, these are merely intended to describe the technical idea of the present disclosure and are not intended to limit the meanings thereof or the scope of the present disclosure described in the claims. Therefore, those of ordinary skill in the art will appreciate that various modifications and other equivalent embodiments are possible from the embodiments. Therefore, the technical scope of the present disclosure should be defined by the technical spirit of the appended claims.

## Claims

1. A breast implant comprising:
a first shell having an inner space in which a first filling material is accommodated;
a second shell surrounding the first shell and having an inner space in which a second filling material is accommodated;
a third shell surrounding the second shell and having an inner space in which a third filling material is accommodated; and
an inlet providing a space through which the first filling material, the second filling material, and the third filling material are injected into the first shell, the second shell, and the third shell, respectively.

2. The breast implant of claim 1, wherein:
a density of the first filling material is higher than a density of the second filling material; and
the density of the second filling material is higher than a density of the third filling material.

3. The breast implant of claim 1, wherein:
a viscosity of the first filling material is higher than a viscosity of the second filling material; and
the viscosity of the second filling material is higher than a viscosity of the third filling material.

4. The breast implant of claim 1, wherein:
a cohesive force of the first filling material is greater than a cohesive force of the second filling material; and
the cohesive force of the second filling material is greater than a cohesive force of the third filling material.

5. The breast implant of claim 2, wherein:
the first filling material is a first silicone;
the second filling material is a second silicone whose density is lower than the first silicone; and
the third filling material is a third silicone whose density is lower than the second silicone.

6. The breast implant of claim 2, wherein:
the first filling material is a first silicone;
the second filling material is a second silicone whose density is lower than the first silicone; and
the third filling material is a saline solution whose density is lower than the second silicone.

7. The breast implant of claim 1, wherein the inlet is provided at a portion where the first shell, the second shell, and the third shell are combined.

8. The breast implant of claim 7, wherein the inlet includes:
a first inlet providing a space through which the first filling material is injected into the first shell;
a second inlet surrounding the first inlet and providing a space through which the second filling material is injected into the second shell; and
a third inlet surrounding the second inlet and providing a space through which the third filling material is injected into the third shell.

9. The breast implant of claim 1, wherein the inlet corresponds to an areolar portion of the human body.

10. The breast implant of claim 1, further comprising a sealing member blocking the inlet.

11. The breast implant of claim 1, wherein:
the first shell includes a first filling portion accommodating the first filling material and a first hanging portion connected to the first filling portion and providing the first filling portion in a hanging form inside the second shell; and
the second shell includes a second filling portion accommodating the second filling material and a second hanging portion connected to the second filling portion and providing the second filling portion in a hanging form inside the third shell.

12. A breast implant comprising:
a first shell having an inner space in which a first filling material is accommodated;
a second shell surrounding the first shell and having an inner space in which a second filling material is accommodated; and
an inlet providing a space through which the first filling material and the second filling material are injected into the first shell and the second shell, respectively,
wherein a density of the first filling material is higher than a density of the second filling material.

13. The breast implant of claim 12, wherein:
the first filling material is a silicone having a first density; and
the second filling material is a silicone having a second density lower than the first density.

14. The breast implant of claim 12, wherein:
the first filling material is a silicone having a first density; and
the second filling material is a saline solution having a second density lower than the first density.

15. The breast implant of claim 12, wherein a viscosity of the first filling material is higher than a viscosity of the second filling material.

16. The breast implant of claim 12, wherein a cohesive force of the first filling material is greater than a cohesive force of the second filling material.

17. The breast implant of claim 12, wherein:
the inlet corresponds to an areolar portion of the human body; and
the inlet includes a first inlet providing a space through which the first filling material is injected into the first shell and a second inlet surrounding the first inlet and providing a space through which the second filling material is injected into the second shell.

18. The breast implant of claim 12, wherein the first shell includes:
a first filling portion accommodating the first filling material; and
a first hanging portion connected to the first filling portion and providing the first filling portion in a hanging form inside the second shell,
wherein, when the breast implant is placed on a floor with the inlet facing upward, a horizontal cross-sectional area of the first hanging portion is smaller than a horizontal cross-sectional area of the first filling portion.
